# EUROPÄISCHE PATENTSCHRIFT

(11) **EP 1 349 863 B1**
(45) Veröffentlichungstag und Bekanntmachung des Hinweises auf die Patenterteilung: **07.07.2004**
(21) Anmeldenummer: 01986868.6
(22) Anmeldetag: 12.12.2001
(51) Int. Cl.: C07F 9/572, C07F 9/6568, C07F 15/00, C07C 45/50, C07B 31/00

(54) **NEUE N-PHENYLPYRROLBISPHOSPHANVERBINDUNGEN UND DEREN METALLKOMPLEXE**
NOVEL N-PHENYL-PYRROL BISPHOSPHANE COMPOUNDS AND THE METAL COMPLEXES OF THE SAME
COMPOSES N-PHENYLE PYRROLE BISPHOSPHANE ET LEURS COMPLEXES METALLIQUES

(30) Priorität: 10.01.2001 DE 10100708
(43) Veröffentlichungstag der Anmeldung: 08.10.2003
(73) Patentinhaber: Oxeno Olefinchemie GmbH, 45772 Marl (DE)
(72) Erfinder: RÖTTGER, Dirk, 45657 Recklinghausen (DE); BELLER, Matthias, 18211 Ostseebad Nienhagen (DE); JACKSTELL, Ralf, 27472 Cuxhaven (DE); HELLER, Detlef, 18334 Dettmannsdorf (DE); DREXLER, Hans-Joachim, 18057 Rostock (DE); KLEIN, Holger, 18069 Rostock (DE); WIESE, Klaus-Diether, 45721 Haltern (DE)
(86) Internationale Anmeldenummer: PCT/EP2001/014619
(87) Internationale Veröffentlichungsnummer: WO 2002/055528

(56) Entgegenhaltungen:
- EP-A- 0 104 375
- WO-A-92/16536
- WO-A-99/52915
- DAI X ET AL: "Synthesis of 2-heterosubstituted quinazolinone atropisomeric phosphine ligands by direct lithiation of a 2-unsubstituted quinazolinone system" TETRAHEDRON: ASYMMETRY, ELSEVIER SCIENCE PUBLISHERS, AMSTERDAM, NL, Bd. 10, Nr. 1, 15. Januar 1999 (1999-01-15), Seiten 25-29, XP004156858 ISSN: 0957-4166
- DREXLER H-J ET AL: "Part III. COD versus NBD precatalysts. Dramatic difference in the asymmetric hydrogenation of prochiral olefins with five-membered diphosphine Rh-hydrogenation catalysts" JOURNAL OF ORGANOMETALLIC CHEMISTRY, ELSEVIER-SEQUOIA S.A. LAUSANNE, CH, Bd. 621, Nr. 1-2, 1. März 2001 (2001-03-01), Seiten 89-102, XP004231241 ISSN: 0022-328X

## Beschreibung

Die vorliegende Erfindung betrifft N-Phenylpyrrolbisphosphane und deren Metallkomplexe, deren Herstellung und Verwendung in katalytischen Reaktionen.

Phosphorhaltige Verbindungen sind als Liganden für Metalle in katalytischen Reaktionen schon lange bekannt. Über die Art des Liganden können Aktivität und Selektivitäten (Chemoselektivität, Regioselektivität, Diastereoselektivität, Enantioselektivität etc.) in weitem Rahmen beeinflusst werden. Dabei sind zweizähnige Liganden einzähnigen Liganden oftmals weit überlegen.

So beschreiben US 4 694 109 und US 4 879 416 Bisphosphinliganden und ihren Einsatz in der Hydroformylierung von Olefinen bei niedrigen Synthesegasdrücken. Besonders bei der Hydroformylierung von Propen werden mit Liganden dieses Typs hohe Aktivitäten und hohe n/i-Selektivitäten erreicht. In WO 95/30680 werden zweizähnige Phosphinliganden und ihr Einsatz in der Katalyse, unter anderem auch in Hydroformylierungsreaktionen, offen gelegt. Ferrocenverbrückte Bisphosphine werden beispielsweise in den Patentschriften US 4 169 861, US 4 201 714 und US 4 193 943 als Liganden für Hydroformylierungen beschrieben.

In asymmetrischen Hydrierungen werden vielfach chirale Bisphosphine sehr erfolgreich eingesetzt. Ein bekanntes Beispiel ist der Ligand BINAP (2,2'-Bis(diphenylphosphino)-1,1'-binaphthyl), der sogar kommerziell erhältlich ist. Dieser Ligand wurde auch in vielen anderen metallkatalysierten Reaktionen eingesetzt, beispielsweise in der Heck Reaktion.

In der Fachliteratur findet sich eine Vielzahl an Veröffentlichungen, die den Einsatz von Liganden in Verbindung mit Metallen als Katalysatoren beschreiben. Einen guten Überblick über den aktuellen Stand des Einsatzgebietes für Übergangsmetallkatalysatoren in der Organischen Synthese findet sich in *Matthias Beller, Carsten Bolm* (Ed.) Transition Metals for Organic Synthesis", Wiley-VCH, Weinheim, New-York, Chichester, Brisbane, Singapore, Toronto, **1998**, *Vol. 1&2.* Beispiele für Katalysatoren und ihre Einsatzgebiete, gängige großtechnische Prozesse usw. finden sich in *B. Cornils, W. A. Herrmann (Ed.),* "Applied Homogeneous Catalysis with Organometallic Compounds", VCH, Weinheim, New-York, Basel, Cambridge, Tokyo, **1996**, *Vol. 1&2.*

Trotz der Fülle an bereits bekannten Systemen gibt es keine "Standardlösungen" für Metall-katalysierte Reaktionen. Je nach Substrat und Reaktion müssen die Bedingungen neu optimiert werden. Zudem ist der Aufwand zur Synthese einiger Liganden relativ hoch, so dass ihr Einsatz in technischen Prozessen nicht rentabel ist.

Es besteht daher ein Bedarf an weiteren einfach zugänglichen Ligandsystemen für Metall-katalysierte Reaktionen.

Es wurde gefunden, dass N-Phenylpyrrolbisphosphane mit der allgemeinen Struktur I 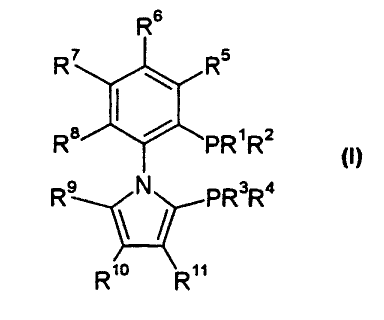 einfach hergestellt werden können und als Liganden in Metall-katalysierten Reaktionen geeignet sind.

Gegenstand der vorliegenden Erfindung sind daher N-Phenylpyrrolbisphosphane der allgemeinen Formel I 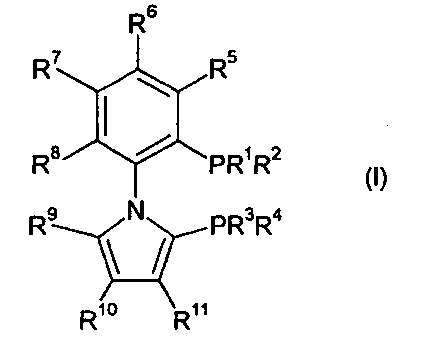 mit
R¹, R², R³, R⁴ = aliphatischer, cycloaliphatischer oder aromatischer Kohlenwasserstoffrest mit 1 bis 25 Kohlenstoffatomen, wobei R¹ und R² bzw. R³ und R⁴ über eine oder mehrere kovalente Bindungen verknüpft sein können,
R⁵, R⁶, R⁷, R⁸, R⁹, R¹⁰, R¹¹ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, wobei R⁵ bis R¹¹ eine gleiche oder unterschiedliche Bedeutung besitzen und kovalent miteinander verknüpft sein können, -Si(CH₃)₃, F, Cl, Br, I, -CF₃, -OR¹², -COR¹², -CO₂R¹², -CO₂M, -SR¹², -SO₂R¹², -SOR¹², -SO₃R¹², -SO₃M -SO₂NR¹²R¹³, NR¹²R¹³, N⁺R¹²R¹³R¹³, N=CR¹²R¹³, NH₂, wobei R¹², R¹³ = H, substituierter oder unsubstituierter, aliphatischer oder aromatischer Kohlenwasserstoffrest mit 1 bis 25 Kohlenstoffatomen, mit gleicher oder unterschiedlicher Bedeutung und M = Alkalimetall-, Erdalkalimetall-, Ammonium-, Phosphoniumion ist.

Spezielle Ausführungsformen der erfindungsgemäßen N-Phenylpyrrolbisphosphane betreffen Phosphine der Formeln II, III, IV und V 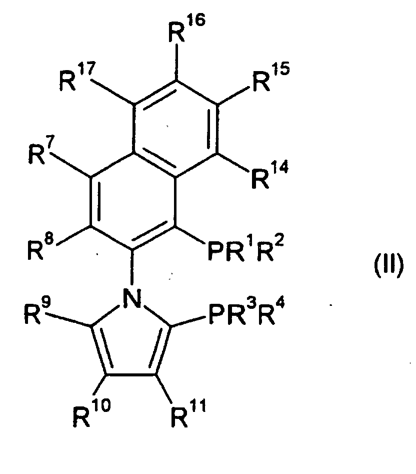 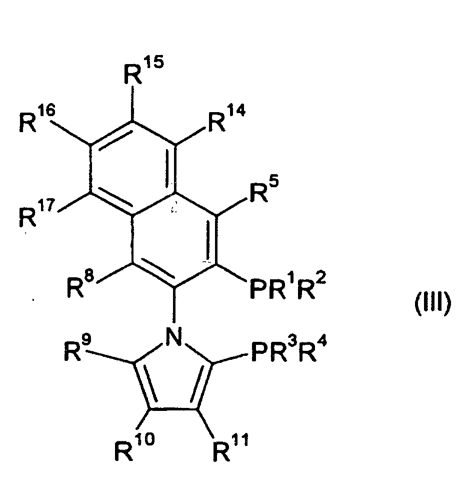 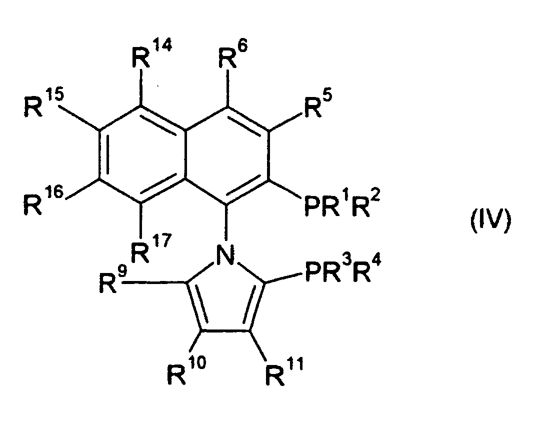 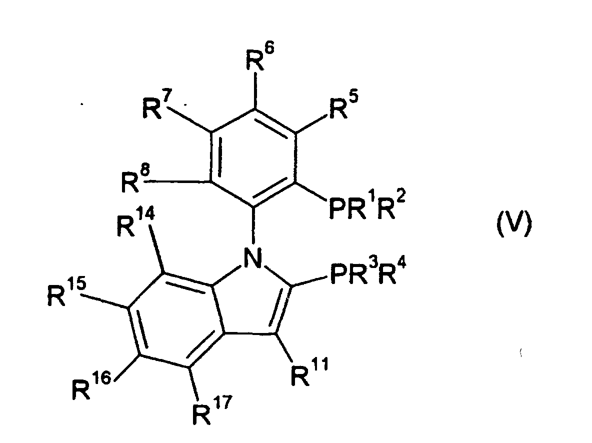 worin jeweils die Restpaare R⁵ und R⁶, R⁶ und R⁷, R⁷ und R⁸ oder R⁹ und R¹⁰ gemeinsam für einen annelierten, ggf. ebenfalls substituierten Aromaten stehen.

Die Substituenten des zusätzlichen aromatischen Systems R¹⁴, R¹⁵, R¹⁶ und R¹⁷ können H, ein aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen sein, wobei R¹⁴ bis R¹⁷ eine gleiche oder unterschiedliche Bedeutung besitzen und kovalent miteinander verknüpft sein können, F, Cl, Br, I, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³, NR¹²R¹³, N⁺R¹²R¹³R¹³, N=CR¹²R¹³, NH₂, wobei R¹² , R¹³ = H, substituierter oder unsubstituierter, aliphatischer oder aromatischer Kohlenwasserstoffrest mit 1 bis 25 Kohlenstoffatomen, mit gleicher oder unterschiedlicher Bedeutung und M = Alkalimetall-, Erdalkalimetall-, Ammonium-, Phosphoniumion bedeuten.

R¹, R², R³, R⁴, R⁵, R⁶, R⁷, R⁸, R⁹, R¹⁰ und R¹¹ besitzen in den Formeln II, III, IV und V die in Anspruch 1 bzw. für Formel I genannten Bedeutungen, wobei jeweils R¹ bis R⁴ und R⁵ bis R¹¹ eine gleiche oder unterschiedliche Bedeutung besitzen können.

Die aliphatischen, cycloaliphatischen oder aromatischen Kohlenwasserstoffreste können Heteroatome, beispielweise Stickstoff, Sauerstoff, oder Schwefel, enthalten und gegebenenfalls einen oder mehrere Substituenten, beispielsweise Halogenatome, tragen. Beispiele für R¹-R⁴ sind Phenyl, m-Sulfonatophenyl, p-Fluorphenyl, o-Fluorphenyl, m-Fluorphenyl, p-Methoxyphenyl, o-Methoxyphenyl, m-Methoxyphenyl, 1-Naphthyl, 2-Naphthyl, Methyl, Ethyl, n-Propyl, i-Propyl, n-Butyl, s-Butyl, i-Butyl, Hexyl, Cyclohexyl, Adamantyl. Beispiele in denen R¹ und R² bzw. R³ und R⁴ eine oder mehrere kovalente Bindung aufweisen sind 1,1'-Diphenyl-2,2'-diyl, Cyclooctan-1,5-diyl. Bevorzugt stehen R¹ bis R⁴ für substituierte oder unsubstituierte Phenyl-, Cyclohexyl- und Adamantylgruppen.

Bei einer eingeschränkten Rotation um die N-Phenylbindung kommt es bei einigen der erfindungsgemäßen Verbindungen aufgrund einer axialen Chiralität zur Ausbildung von Enantiomeren, bei zusätzlicher Chiralität in den Resten R¹-R¹¹ zu Diastereomeren.

In besonderen Ausführungsformen der Erfindung sind die N-Phenylpyrrolbisphosphane chiral, bevorzugt sind einer oder mehrere der Reste R' bis R¹¹ chiral. Besonders bevorzugt werden dabei chirale Substituenten für R¹-R⁴. Beispiele für solche chiralen Gruppen sind Menthyl, Camphyl, 1,1'-Binaphth-2-yl, Hexan-2,5-diyl.

Die Verwendung von einem oder mehreren chiralen Resten in R' bis R" bietet u.a. den Vorteil, dass bei Auftreten einer axialen Chiralität (N-Phenylbindung als Chiralitätsachse) die Diastereomere leicht getrennt werden können.

Gegenstand der Erfindung sind sowohl Mischungen einzelner Enantiomere bzw. Diastereomere der erfindungsgemäßen N-Phenylpyrrolbisphosphanliganden als auch die einzelnen Enantiomere bzw. Diastereomere selbst und ihr Einsatz in enantioselektiven oder diastereoselektiven Katalysereaktionen. Gegenstand der vorliegenden Erfindung sind weiterhin N-Phenylpyrrolbisphosphanmetallkomplexe, enthaltend ein Nebengruppenmetall der 1., 2., 3., 4., 5., 6., 7. oder 8 Nebengruppe des Periodensystems, die Elemente der Lanthanoiden und/oder Actinoiden und ein oder mehrere N-Phenylpyrrolbisphosphane der Formeln I, II, III, IV oder V. Die Substituenten (R¹-R¹⁷) dieser N-Phenylpyrrolbisphosphane besitzen dabei die bereits genannten Bedeutungen. Bevorzugt eingesetzte Metalle sind Ti, Zr, Cr, Mo, W, Mn, Re, Fe, Ru, Os, Co, Rh, Ir, Ni, Pd, Pt, Cu, Ag und Zn. Besonders bevorzugt sind die Metalle der 8. Nebengruppe des Periodensystems.

Die Ausführungen über alle Ausführungsformen der N-Phenylpyrrolbisphosphane der Formeln I bis V gelten entsprechend für die Metallkomplexe. Sind die N-Phenylpyrrolbisphosphanliganden in der beschriebenen Weise chiral, so können die Metallkomplexe einen, mehrere oder ausschließlich chirale N-Phenylpyrrolbisphosphenliganden enthalten.

Im Folgenden werden repräsentative Beispiele von Liganden nach den allgemeinen Formeln I, II, III, IV und V im Sinne dieser Erfindung dargestellt, ohne den Schutzbereich der vorliegenden Erfindung zu beschränken.

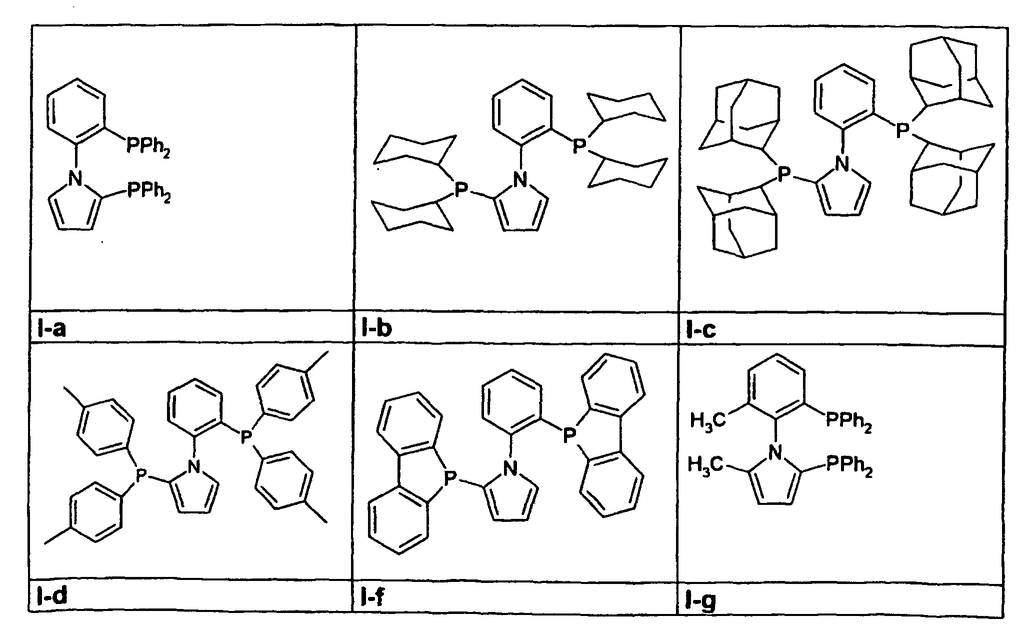

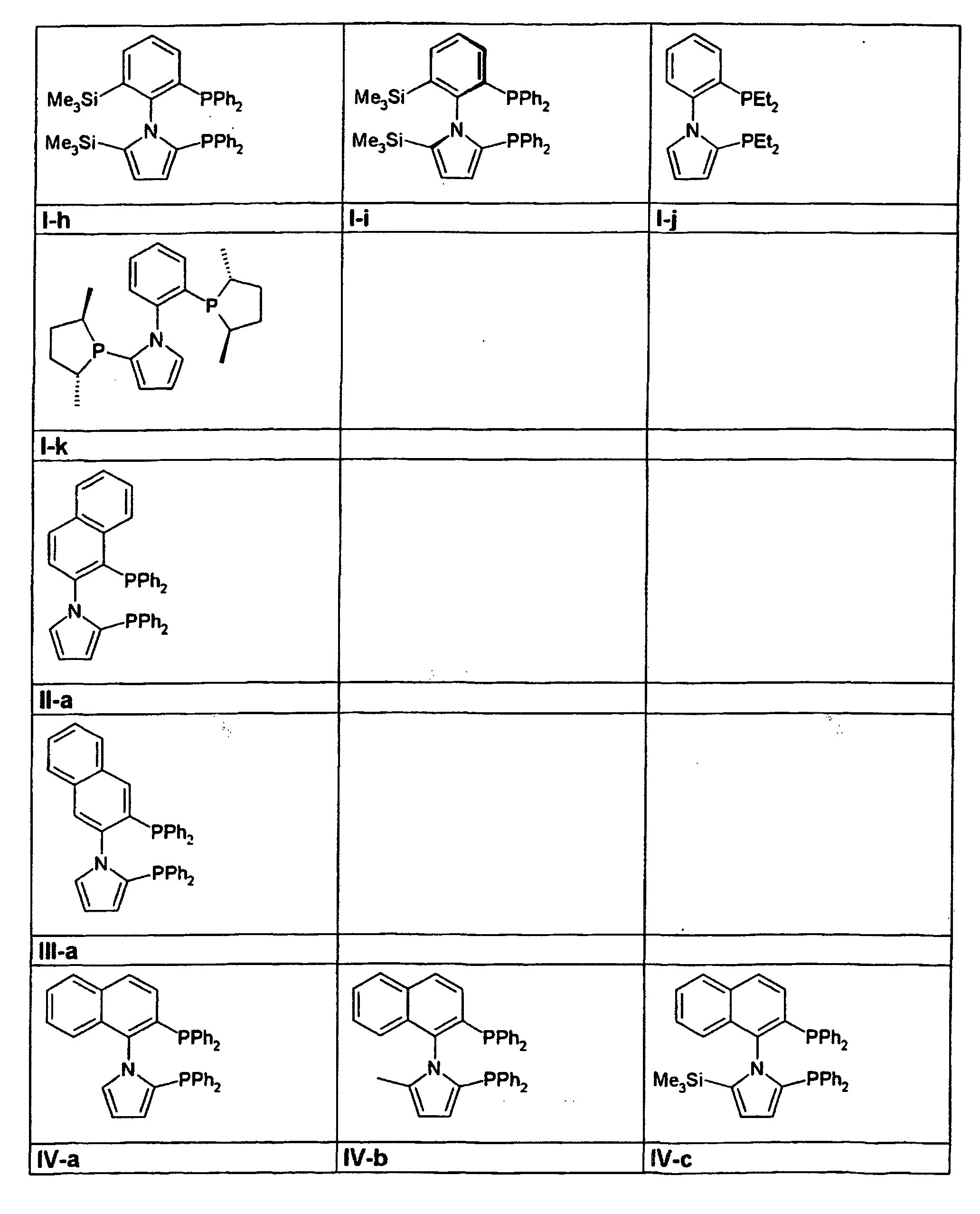

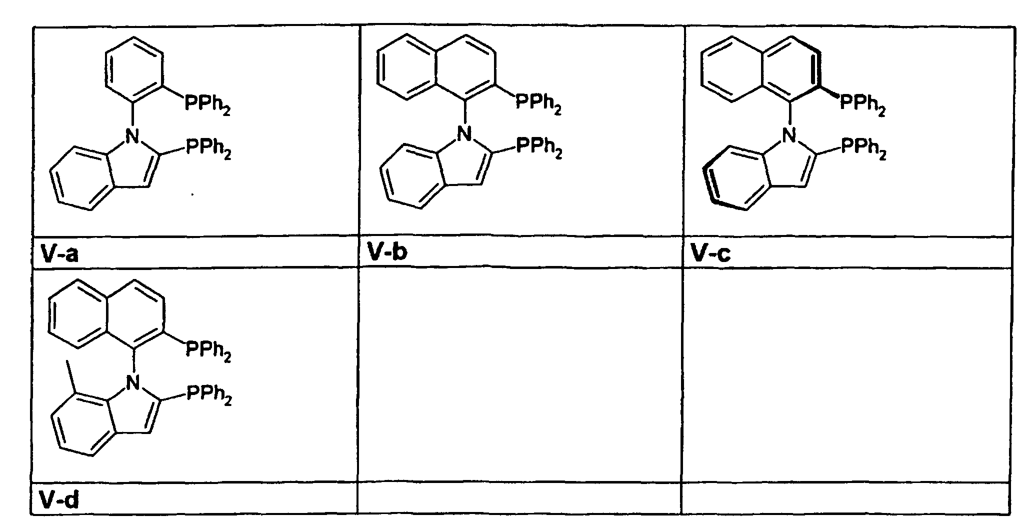

Die erfindungsgemäßen N-Phenylpyrrolbisphosphane können über verschiedene Synthesewege erhalten werden. Ein einfacher Zugang ist die doppelte Metallierung von N-Phenylpyrrolen und die nachfolgende Umsetzung mit der Phosphorkomponente (R³ = R¹, R⁴ = R²).

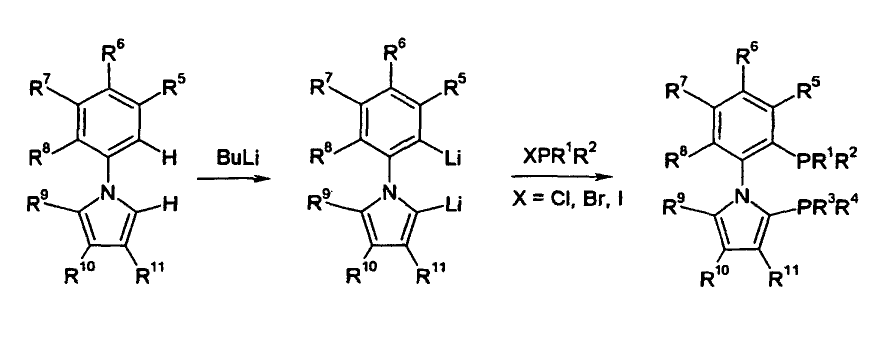

Die Reste R⁵ - R¹¹ können auch nach Anbindung der Phosphorgruppen eingebracht werden. Dieser Weg wurde beispielsweise bei der Synthese von Verbindung I-h beschritten:

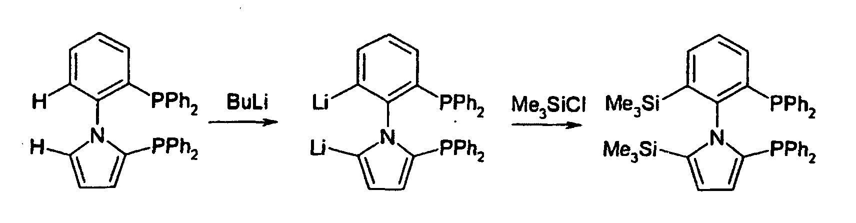

Racemisch anfallende Liganden können nach literaturbekannten Routen in die optischen Isomere aufgetrennt werden, beispielsweise chromatographisch an chiralen Trägern oder als Diastereomere nach Umsetzung mit chiralen Hilfsstoffen und anschließender Spaltung.

Die erfindungsgemäßen N-Phenylpyrrolbisphosphane der Formeln I, II, III, IV und V zeichnen sich durch eine hohe Hydrolysestabilität aus. Sie sind in den gängigen organischen Solventien ausreichend gut löslich. Falls eine oder mehrere Gruppen R1 bis R11 oder R14 bis R17 einen stark polaren Rest trägt oder einer oder mehrere der Substituenten R1 bis R4 einen stark polaren Rest tragen (beispielsweise eine Sulfonsäuregruppe) nimmt die Löslichkeit in unpolaren organischen Lösungsmitteln ab, gleichzeitig steigt aber die Löslichkeit in polaren Lösungsmitteln, beispielsweise Wasser, an. Die erfindungsgemäßen N-Phenylpyrrolbisphosphane bzw. deren Metallkomplexe können daher auch in Wasser oder polaren organischen Lösemitteln löslich sein.

Aufgrund ihres hohen Molekulargewichts besitzen die erfindungsgemäßen N-Phenylpyrrolbisphosphane eine geringe Flüchtigkeit. Sie können daher einfach von leichter flüchtigen Reaktionsprodukten abgetrennt werden.

Ein weiterer Gegenstand der Erfindung ist die Verwendung der N-Phenylpyrrolbisphosphane bzw. deren Metallkomplexe in Hydrierungen, Isomerisierungen, Carbonylierungen, Carboxylierungen, Hydroformylierungen, Hydrocyanierungen, Cyclopropanierungen, C-C-Kupplungen, Oligomerisierungen oder Polymerisationen.

Die erfindungsgemäßen N-Phenylpyrrolbisphosphane der Formeln I, II, III, IV und V sind geeignete Bausteine für die Herstellung von Komplexen mit Metallen der 1 bis 8. Nebengruppe des Periodensystems, der Lanthanoide und/oder Actionide. Insbesondere mit Metallen der 6. bis 8. Nebengruppe können diese Komplexe als Katalysatoren für Hydrierungen, Isomerisierungen, Carbonylierungen, Carboxylierungen, Hydroformylierungen, Hydrocyanierungen, Cyclopropanierungen, C-C-Kupplungsreaktionen, Oligomerisationen und Polymerisationen eingesetzt werden. Bevorzugte Einsatzgebiete sind Hydrierungen, Hydroformylierungen und C-C-Kupplungsreaktionen.

Bei Einsatz der erfindungsgemäßen N-Phenylpyrrolbisphosphane gemäß den Formeln I bis V, ggf. als Metallkomplex, in Hydrierungen werden Metalle der 8. Nebengruppe des Periodensystems bevorzugt. Als Substrate werden in den Hydrierungen bevorzugt Verbindungen mit Doppelbindungen eingesetzt, beispielsweise C=C-, C=O oder C=N-Doppelbindungen, die zur Einfachbindung hydriert werden. Beispiele für Hydrierungen finden sich zum Beispiel in *Matthias Beller, Carsten Bolm* (Ed.) "Transition Metals for Organic Synthesis", Wiley-VCH, Weinheim, New-York, Chichester, Brisbane, Singapore, Toronto, **1998**, *Vol.* 2, Seite 3-80.

Bei Einsatz der erfindungsgemäßen N-Phenylpyrrolbisphosphane gemäß den Formeln I bis V, ggf. als Metallkomplex, in Hydroformylierungen werden Metalle der 8. Nebengruppe des Periodensystems bevorzugt. Besonders bei Einsatz von Rhodium als Katalysatormetall ergeben sich hohe katalytische Aktivitäten in den Hydroformylierungen. Als Edukte werden in diesen Hydroformylierungen bevorzugt Olefine mit 2 bis 25 Kohlenstoffatomen eingesetzt, beispielsweise Butene (1-Buten, 2-Buten, i-Buten), Octene (1-Octen, Dibutene), Dodecene (Tributene). Bei Einsatz der erfindungsgemäßen N-Phenylpyrrolbisphosphane gemäß den Formeln I bis V, ggf. als Metallkomplex, in C-C-Kupplungsreaktionen werden Metalle der 8. Nebengruppe des Periodensystems bevorzugt, ganz besonders der Nickel und Palladium. Beispiele für C-C-Kupplungsreaktionen finden sich zum Beispiel in *Matthias Beller, Carsten Bolm* (Ed.) "Transition Metals for Organic Synthesis", Wiley-VCH, Weinheim, New-York, Chichester, Brisbane, Singapore, Toronto, **1998**, *Vol. 1,* Seite 208-240.

In den Metall-katalysierten Reaktionen werden entweder die N-Phenylpyrrolbisphosphanmetallkomplexe, ggf. mit zusätzlichem, freiem Ligand, oder die N-Phenylpyrrolbisphosphane und eine Metallverbindung, aus der sich unter den Reaktionsbedingungen der Katalysatorkomplex mit den N-Phenylpyrrolbisphosphanen bildet, eingesetzt.

Bei Einsatz der erfindungsgemäßen N-Phenylpyrrolbisphosphanverbindungen und/oder ihrer Metallkomplexe in Metall-katalysierten Reaktionen liegt das Verhältnis von Ligand zu Metall (mol/mol) bei 1 : 2 bis 200 : 1, bevorzugt bei 1 : 1 bis 1 : 50, besonders bevorzugt bei 1 : 1 bis 1 : 20. Ligand bzw. Ligand-Metall-Komplex liegen dabei in der Regel homogen gelöst in einer oder mehreren der vorhandenen flüssigen Phasen vor. Auch ein Einsatz der N-Phenylpyrrolbisphosphane in "Supported aqueous phase"-Katalysatoren ist möglich.

Die folgenden Beispiele sollen die Erfindung erläutern, nicht aber ihren Anwendungsbereich beschränken, der sich aus den Patentansprüchen ergibt.

### Beispiele

Alle Operationen werden unter Argon mittels Schlenktechnik mit getrockneten und entgasten Lösungsmitteln durchgeführt.

### Beispiel 1

### 1-(2-Diphenylphosphinophenyl)pyrrol-2-diphenylphosphin (I-a, JaPHOS)

1.52 g (10.6 mmol ) N-Phenylpyrrol werden in einem 250 ml Dreihalskolben (versehen mit Magnetrührung) in 50 ml Et₂O gelöst und mit 2.5 g (3.2 ml) TMEDA (Tetramethylethylendiamin) versetzt. Bei 25 °C werden anschließend 13.6 ml 1.6 molarer n-Butyllithiumlösung (21.2 mmol ) zugesetzt. Der Reaktionsansatz wird 12 h bei Raumtemperatur gerührt. Diese Reaktionslösung I wird in einen Tropftrichter überführt. In einem 250 ml Dreihalskolben werden 4.68 g (21.23 mmol) Diphenylchlorphosphin mit 50 ml Ether gemischt. Diese Lösung II wird auf 0 °C gekühlt. Zu dieser Lösung II wird bei 20°C langsam die Lösung I mit dem dilithiiertem N-Phenylpyrrol zugetropft. Anschließend wird noch eine 1 h bei Raumtemperatur gerührt. Die Reaktionslösung wird mit 20 ml Wasser versetzt und 10 min gerührt. Es werden die Phasen getrennt und die organische Phase über Natriumsulfat 12 h getrocknet. Anschließend wird im Vakuum eingeengt und der Rückstand in 20 ml Toluol gelöst. Diese Lösung wird mit 100 ml Hexan vorsichtig überschichtet. Das Zielprodukt kristallisiert innerhalb eines Tages aus. Es wird filtriert und im Vakuum getrocknet.
Ausbeute: 4.88 g (90% d. Theorie); M = 511.54 g/Mol
³¹P-NMR:(δ[ppm], J[Hz], CDCl₃): -17.5 d, J_{PP} = 18; -30.7 d, J_{PP} = 18
¹H-NMR: (δ[ppm], J[Hz], CDCl₃): 6.01 d,d, J = 3.6, J = 3.6 (1 H);6.11 d, d, J = 3.57, J = 3.57 (1 H); 6.58 m (1 H), 7.0 m(4H); 7.1-7.3 m (20H)
¹³C-NMR: (δ[ppm], J[Hz], CDCl₃): 108.9 s; 118.9s; 128-129 m; 130.0 d, J = 3; 133-134 m;
34.8 d, J = 2.8; 136.9 s; 137.0 d, J = 2; 137.3 d, J = 13.3; 137.7 d, ¹Jpc = 17.16; 137.8 d, ¹J_{PC} = 15.2; 144.4 d, ¹J_{PC} = 26; 144.7 d, ¹J_{PC} = 26
MS, m/z(%): 511 (1)[M⁺], 434(1)[M⁺-Ph], 326(100)[M⁺-PPh₂], 249(10)[M⁺-PPh₂-Ph], 183(15)[PPh₂], 172(8)[M⁺-PPh₂-2Ph]; EA : ber:C:79.8, H:5.32, N:2.7; gef:C:79.9, H:5.5, N:2.5

### Beispiel 2

### 1-(2-Dicyclohexylphosphinophenyl)pyrrol-2-dicyclohexylphosphin (I-b, Cyc-JaPHOS)

0.43g (3.04 mmol) N-Phenylpyrrol werden in einem 50 ml Dreihalskolben (versehen mit Magnetrührung) in 20 ml Et₂O gelöst und mit 0.704g (0.91 ml) TMEDA (Tetramethylethylendiamin) versetzt. Bei 25 °C werden anschließend 3.8 ml 1.6 molarer n-Butyllithiumlösung (6.06 mmol) zugesetzt. Der Reaktionsansatz wird 12h bei 25 °C gerührt. Diese Reaktionslösung I wird in einen Tropftrichter überführt. In einem 100 ml Dreihalskolben werden 1.41 g (6.06 mmol) Dicyclohexylchlorphosphin mit 20 ml Et₂O gemischt. Diese Lösung II wird auf 0 °C gekühlt. Zur Lösung II wird bei 0 °C die Lösung I mit dem dilithiiertem N-Phenylpyrrol zugetropft. Anschließend wird 1 h bei 25 °C gerührt. Die Reaktionslösung wird mit 20 ml Wasser versetzt und 10 min gerührt. Es werden die Phasen getrennt und die organische Phase über Natriumsulfat 12 h getrocknet. Anschließend wird im Vakuum eingeengt und der Rückstand in 200 ml heißem Ethanol gelöst. Das Zielprodukt kristallisiert innerhalb eines Tages aus. Es wird filtriert und im Vakuum getrocknet.
Ausbeute: 1.2 g (74% d. Theorie); M = 535.73 g/Mol
³¹P-NMR:(δ[ppm], J[Hz], CDCl₃): 15.9 d, J_{PP} = 6.94; -28.1 d, J_{PP} = 6.94
¹H-NMR: (δ[ppm], J[Hz], CDCl₃): 0.9-1.2 m(21 H); 1.5-1.8 m (23H); 6.27 d,d, J = 2.57, J = 2.58 (1H); 6.45 d,d, J = 1.59, J = 1.58(1 H); 6.7 m(1 H); 7.1 m(1 H); 7.2-7.3 m(2H); 7.4-7.5 m (1 H)
¹³C-NMR: (δ[ppm], J[Hz], CDCl₃): 26.4 d, J = 10.5; 26.7 d, J = 8.6; 27.0-27.6 m; 28.6 d, J = 5.8; 29.7 d, J = 11.4; 29.8 d, J = 15; 30.5 d, J = 19; 30.9, 31 d,d J = 15; 31.4, 31.45 d,d, J = 16; 34.6 d, J = 26.7; 34.8 d, J =19.1 ; 35.4 d, J = 9.54; 36.1 d, J = 16.2; 107.6 s; 116 d, J = 3.8; 127 s; 127.5 s; 128.1 s; 129.5 d, J = 6.7; 130.0 d,d, J = 3.8; 133.0 d, J = 3.8; 135.4 d, ¹J_{PC} = 22; 147.01, 147.0 d,d, ¹J_{PC} = 24.8
MS, m/z (%): 535 (2)[M⁺], 452 (100) [M⁺-Cy], 369 (7)[M⁺-2Cy], 338(5)[M⁺-PC_{y2}], 204 (7)[M⁺-4C_{y}]; 83 (5)[Cy), EA: ber:C:76.2,H:9.6,N:2.6; gef:C:75.9, H:9.5 N:2.6

### Beispiel 3

### 1-(2-Diadamantylphosphinophenyl)pyrrol-2-diadamantylphosphin (I-c, Ad-JaPHOS)

0.32 g (2.2 mmol) N-Phenylpyrrol werden in einem 50 ml Dreihalskolben (versehen mit Magnetrührung) in 20 ml Et₂O gelöst und mit 0.5 g (0.75 ml) TMEDA (Tetramethylethylendiamin) versetzt. Bei 25 °C werden anschließend 2.8 ml 1.6 molarer n-Butyllithiumlösung (4.4 mmol ) zugesetzt. Der Reaktionsansatz wird 12 h bei 25 °C gerührt. Diese Reaktionslösung I wird in einen Tropftrichter überführt. In einem 100 ml Dreihalskolben werden 1.5 g (4.4 mmol) Diadamantylchlor-phosphin mit 40 ml THF gemischt. Diese Lösung II wird auf 0 °C gekühlt. Zur Lösung II wird bei 0 °C die Lösung I zugetropft. Anschließend wird erwärmt und 36 h im Rückfluß (ca. 60 °C) gerührt. Die Reaktionslösung wird im Vakuum eingeengt, der Rückstand mit 20 ml Toluol gelöst, mit 20 ml Wasser versetzt und 10 min gerührt. Es werden die Phasen getrennt und die organische Phase mit etwas trockenem Natriumsulfat 12 h getrocknet. Nach Filtration werden 100 ml Ethanol zugesetzt. Das Zielprodukt kristallisiert farblos innerhalb eines Tages aus. Es wird filtriert und im Vakuum getrocknet.
Ausbeute: 0.25 g (15.3 % d. Theorie); M = 744.03g/Mol
³¹P-NMR:(δ[ppm], J[Hz], CDCl₃): 17.81 d, J = 5.5 ; 4.75 d, J = 5.5
¹H-NMR: (δ[ppm], J[Hzl, CDCl₃): 1.5-2.0 m (60 H); 6.2 t, J = 3.1 (1H); 6.6 d,d, J = 1.5, J = 3.6 (1 H)
6.9 m (1 H); 7.1 m (1 H); 7.2 m (2 H); 7.8 m (1 H)
MS, mlz (%): 744 (3)[M⁺], 608 (60)[M⁺-Ad), 442 (2)[M⁺-PAd₂], 301(2)[PAd₂], 135 (100)[Ad]

### Beispiele 4 - 7

Allgemeine Arbeitsvorschrift 1 (AAV 1) zur Synthese von Rhodium (I)-NBD bzw.COD-Komplexen mit Cyc-JaPHOS und JaPhOS als Liganden,
1 mmol Rh(NBD)(acac) bzw. Rh(COD) (acac) werden in 10 ml THF gelöst und auf -70 °C gekühlt.

1 mmol Ligand aus Beispiel 1-2 gelöst in 40 ml THF wird innerhalb von 10 Minuten zur Rhodium-Lösung zugetropft. Die Lösung wird unter Magnetrührung auf Raumtemperatur erwärmt und mit 120 µl 54%ige HBF₄ (Lösung in Ether) versetzt. Anschließend wird mit 20 ml Et₂O ausgefällt, filtriert, mit Et₂O gewaschen. Die Ausbeuten betragen über 90 %. Die analytischen Daten der nach AAV 1 dargestellten Verbindungen sind in Tabelle 1 zusammengestellt.

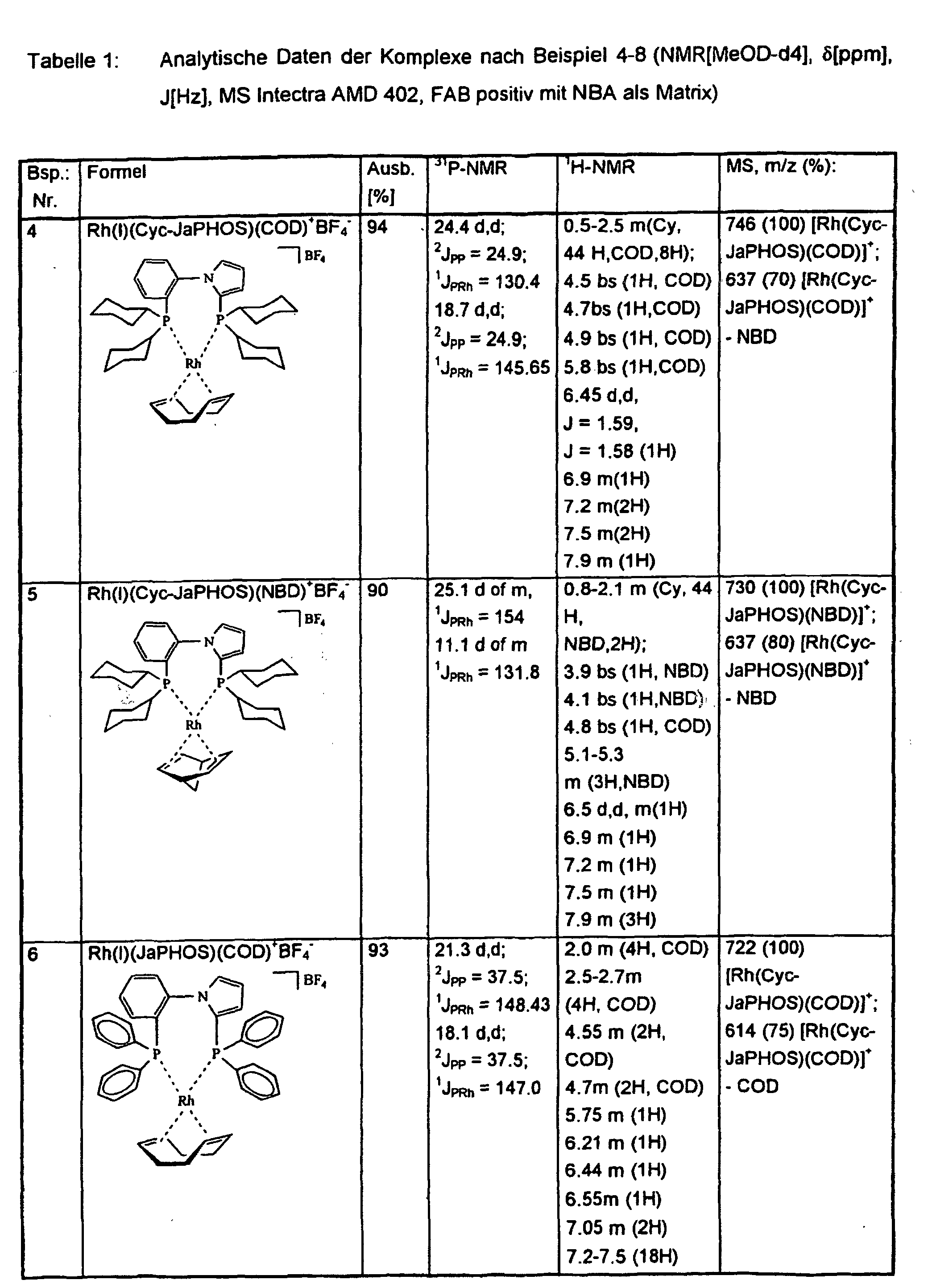

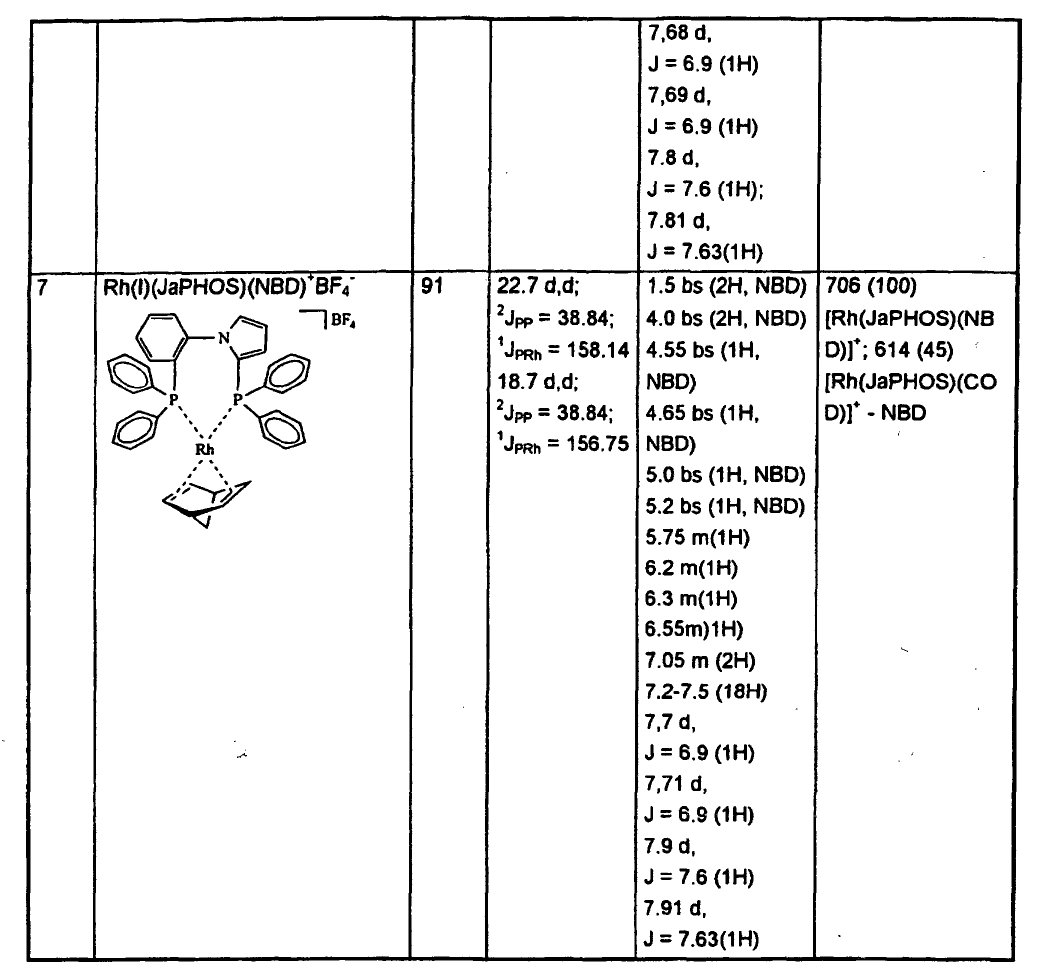

### Beispiel 8: Hydroformylierungsreaktion von 1- oder cis/trans-2-Penten mittels eines Rhodium / JaPHOS-Katalysators

Ein Gemisch von 73 mmol Alken, der entsprechenden Menge Rh(acac)(CO)₂ als Katalysatorvorstufe (0.01 mol% Katalysator 0,0073 mmol = 1,88 mg), der entsprechenden Menge JaPHOS, 2 ml Isooctan als internem Standard sowie 30 ml Anisol als Lösungsmittel werden in einem 100 ml-Autoklaven der Firma Parr (mit Magnetrührung) zur Reaktion gebracht. Der Synthesegasdruck (H₂/Co = 1:1 nach Erreichen der Arbeitstemperatur konstant bei dem angegebenen Wert gehalten. Nach der Reaktionszeit, erfolgter Abkühlung und Entspannung des Autoklaven wird das Reaktionsgemisch gaschromatographisch analysiert (Ergebnisse siehe Tabelle 2)

**Tabelle 2:**

| Hydroformylierungsergebnisse von 1- oder cis/trans-2-Penten mittels eines Rhodium/ JaPHOS-Katalysators | | | | | | | |
|---|---|---|---|---|---|---|---|
| Substrat | Rh [mol%] | Rh:JaPHO S [mol/mol] | T [°C] | P [bar] | T [h] | Ausbeute^{a} [%] | n:iso^{b} |
| 1-Penten | 0.01 | 1:5 | 120 | 50 | 6 | 94 | 47:53 |
| 2-Penten^{c} | 0.01 | 1:5 | 120 | 50 | 6 | 54 | 24:76 |
| 2-Penten ^{c} | 0.01 | 1:5 | 120 | 25 | 6 | 25 | 39:61 |

| | | | | | | | |
|---|---|---|---|---|---|---|---|
| ^{a} Ausbeute = 1-Hexanal+2-Methylpentanal+2-Ethylbutanal, | | | | | | | |
| ^{b}n:iso = 1-Hexanal : 2-Methylpentanal+2-Ethylbutanal, [mol : mol] | | | | | | | |
| ^{c} 2-Penten = cis/trans 2-Penten | | | | | | | |

### Beispiel 9

Allgemeine Arbeitvorschrift zur Hydrierung von Olefinen mittels Katalyse durch Rhodium/ N-Phenylpyrrolyl-Bisphosphinkatalysatoren aus den Beispielen 4-7: (AAV 3)

0.01 mmol (oder weniger) des entsprechenden Komplexes aus Beispiel 4 - 7 werden in einer Glasampulle abgeschmolzen und unter Argon in ein thermostatisiertes Hydriergefäß (versehen mit Magnetrührung) gegeben. Anschließend werden 15 ml Methanol und 1 mmol Substrat zugesetzt. Nach Gasaustausch von Argon durch Wasserstoff wird die Reaktion gestartet in dem die Glasampulle mit dem Präkatalysator zerstört wird. Während der Reaktion werden die Temperatur bei 25 °C und der Wasserstoffdruck bei 1 bar konstant gehalten und der Gasverbrauch mit einer automatischen Registrieranlage gemessen. Es wird die Zeit bis zum vollständigen Umsatzes des Substrates gemessen. Die Produktanalyse erfolgt im Anschluß durch GC. Beispiel siehe Tabelle 3.

**Tabelle 3:**

| Hydrierergebnisse zur Hydrierung von Olefinen mittels Katalyse durch Rhodium/N-Phenylpyrrolyl-Bisphosphinkatalysatoren | | | |
|---|---|---|---|
| Präkatalysator | Substrat | t1 | t2 |
| (mmol) | (mmol) | | |
| Rh(I)(JaPHOS)(COD)⁺BF₄⁻ | COD^{a} | COD→COE ^{b} | COE→COA^{c} |
| (0.01) | (1) | 4.7 h | 3 d |
| Rh(I)(JaPHOS)(NBD)⁺BF4- | NBO^{d} | NBD^{d}→NBE^{e} | NBE^{e}→NBA^{f} |
| (0.01) | (1) | 2.5 min | 40 min |
| Rh(I)(Cyc- | COD^{a} | COD→COE^{b} | COE→COA^{c} |
| JaPHOS)(COD)⁺BF₄⁻ (0.01) | (1) | 100 min | 10 min |
| Rh(I)(Cyc-JaPHOS)(NBD)⁺BF₄⁻ | NBD^{d} | NEBD^{d}→NBE^{e} | NBE^{e}→NBA^{f} |
| (0.0007) | (1) | 2.5 min | 40 min |

| | | | |
|---|---|---|---|
| ^{a} =1,4-Cyclooctadien, | | | |
| ^{b} = Cycloocten, | | | |
| ^{c}= Cyclooctan, | | | |
| ^{d} = 2,5-Norbornadien, | | | |
| ^{e} = 2-Norbomen, | | | |
| ^{f} = Norbornan | | | |

## Patentansprüche

1. N-Phenylpyrrolbisphosphane der Formel I 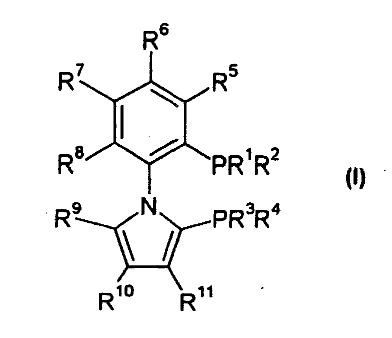 mit
R¹, R², R³, R⁴ = aliphatischer, cycloaliphatischer oder aromatischer Kohlenwasserstoffrest mit 1 bis 25 Kohlenstoffatomen, wobei R¹ und R² bzw. R³ und R⁴ über eine oder mehrere kovalente Bindungen verknüpft sein können,
R⁵, R⁶, R⁷, R⁸, R⁹, R¹⁰, R¹¹ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, wobei R⁵ bis R¹¹ eine gleiche oder unterschiedliche Bedeutung besitzen und kovalent miteinander verknüpft sein können, F, Cl, Br, l, -Si(CH₃)₃, -CF₃, -OR¹², -COR¹², -CO₂R¹², -CO₂M, -SR¹², SO₂R¹² -SOR¹², -SO₃R¹², -SO₃M, -SO₂NR¹²R¹³, NR¹²R¹³, N+R¹²R¹³R¹³, N=CR¹²R¹³, NH₂, wobei R¹², R¹³ = H, substituierter oder unsubstituierter, aliphatischer oder aromatischer Kohlenwasserstoffrest mit 1 bis 25 Kohlenstoffatomen, mit gleicher oder unterschiedlicher Bedeutung und M = Alkalimetall-, Erdalkalimetall-, Ammonium-, Phosphoniumion.

2. N-Phenylpyrrolbisphosphane gemäß Anspruch 1,
**dadurch gekennzeichnet,**
**dass** R⁵ und R⁶ aus Formel I gemäß Formel II gemeinsam für einen annelierten Aromaten stehen, 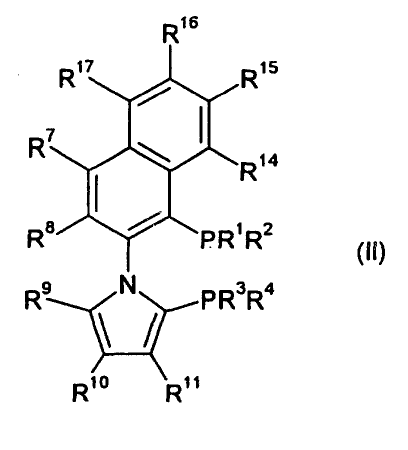 mit
R¹⁴, R¹⁵, R¹⁶, R¹⁷ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, F, Cl, Br, I, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³ NR¹²R¹³, N⁺R¹²R¹³R¹³, N=CR¹²R¹³, NH₂, wobei R¹², R¹³ = H, substituierter oder unsubstituierter, aliphatischer oder aromatischer Kohlenwasserstoffrest mit 1 bis 25 Kohlenstoffatomen, mit gleicher oder unterschiedlicher Bedeutung und M = Alkalimetall-, Erdalkalimetall-, Ammonium-, Phosphoniumion und R¹, R², R³, R⁴, R⁷, R⁸, R⁹, R¹⁰ und R¹¹ die in Anspruch 1 genannten Bedeutungen besitzen.

3. N-Phenylpyrrolbisphosphane gemäß Anspruch 1,
**dadurch gekennzeichnet,**
**dass** R⁶ und R⁷ aus Formel I gemäß Formel III gemeinsam für einen annelierten Aromaten stehen, 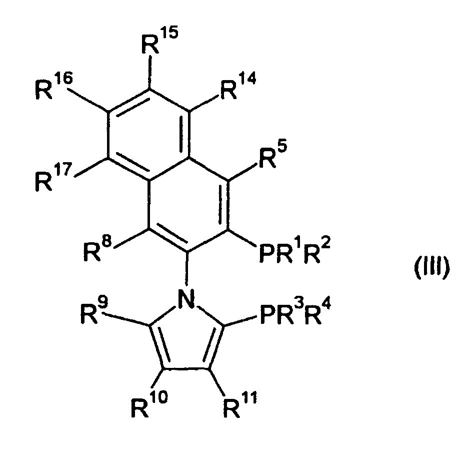 mit
R¹⁴, R¹⁵, R¹⁶, R¹⁷ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, F, Cl, Br, I, -Si(CH₃)₃, -CF₃, -OR¹², -COR¹², -CO₂R¹², -CO₂M, -SR¹², -SOR¹², -SOR¹², SO₃R¹², -SO₃M, -SO₂NR¹²R¹³, NR¹²R¹³, N⁺R¹²R¹³R¹³, N=CR¹²R¹³, NH₂, wobei R¹², R¹³ = H, substituierter oder unsubstituierter, aliphatischer oder aromatischer Kohlenwasserstoffrest mit 1 bis 25 Kohlenstoffatomen, mit gleicher oder unterschiedlicher Bedeutung und M = Alkalimetall-, Erdalkalimetall-, Ammonium-, Phosphoniumion und R¹, R², R³, R⁴, R⁵, R⁸, R⁹, R¹⁰ und R¹¹ die in Anspruch 1 genannten Bedeutungen besitzen.

4. N-Phenylpyrrolbisphosphane nach Anspruch 1,
**dadurch gekennzeichnet,**
**dass** R⁷ und R⁸ aus Formel I gemäß Formel IV gemeinsam für einen annelierten Aromaten stehen, 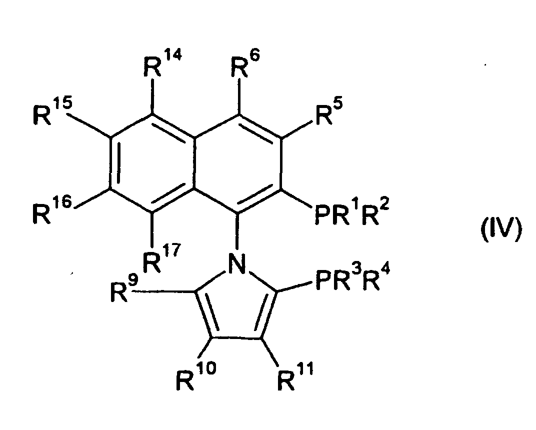 mit
R¹⁴, R¹⁵, R¹⁶, R¹⁷ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, F, Cl, Br, I, -Si(CH₃)₃, -CF₃, -OR¹², -COR¹², -CO₂R¹², CO₂M, -SR¹², -SO₂R¹², -SOR¹², -SO₃R¹², -SO₃M, -SO₂NR¹²R¹³ NR¹²R¹³, N+R¹²R¹³R¹³, N=CR¹²R¹³, NH₂, wobei R¹², R¹³= H, substituierter oder unsubstituierter, aliphatischer oder aromatischer Kohlenwasserstoffrest mit 1 bis 25 Kohlenstoffatomen, mit gleicher oder unterschiedlicher Bedeutung und M = Alkalimetall-, Erdalkalimetall-, Ammonium-, Phosphoniumion und R¹, R², R³, R⁴, R⁵, R⁶, R⁹, R'° und R¹¹ die in Anspruch 1 genannten Bedeutungen besitzen.

5. N-Phenylpyrrolbisphosphane gemäß Anspruch 1,
**dadurch gekennzeichnet,**
**dass** R⁹ und R¹⁰ aus Formel I gemäß Formel V gemeinsam für einen annelierten Aromaten stehen, 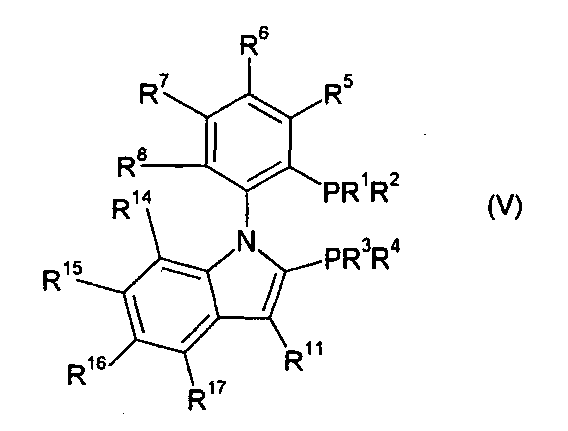 mit
R¹⁴, R¹⁵, R¹⁶, R¹⁷ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohltenstoffatomen, F, Cl, Br, I, -Si(CH₃)₃, -CF₃, -OR¹², -COR¹², -CO₂R¹², -CO₂M, -SR¹², -SOR¹², -SOR¹² -SOR¹², -SO₃M, SO₂NR¹²R¹³, NR¹²R¹³, N⁺R¹²R¹³R¹³, N=CR¹²R¹³, NH₂, wobei R¹², R¹³ = H, substituierter oder unsubstituierter, aliphatischer oder aromatischer Kohlenwasserstoffrest mit 1 bis 25 Kohlenstoffatomen, mit gleicher oder unterschiedlicher Bedeutung und M = Alkalimetall-, Erdalkalimetall-, Ammonium-, Phosphoniumion und R¹, R², R³, R⁴, R⁵, R⁶, R⁷, R⁸ und R¹¹ die in Anspruch 1 genannten Bedeutungen besitzen.

6. N-Phenylpyrrolbisphosphane nach einem der Ansprüche 1 bis 5,
**dadurch gekennzeichnet,**
**dass** die N-Phenylpyrrolbisphosphane chiral sind.

7. N-Phenylpyrrolbisphosphane nach einem der Ansprüche 1 bis 6,
**dadurch gekennzeichnet,**
**dass** einer oder mehrere der Reste R¹-R⁴ oder R¹ und R² bzw. R³ und R⁴ gemeinsam für einen chiralen Rest aus der Gruppe Menthyl, Camphyl, 1,1'-Binaphth-2-yl, Hexan-2,5-diyl stehen.

8. N-Phenylpyrrolbisphosphan-Metallkomplex, enthaltend ein Metall der 1., 2., 3., 4., 5., 6., 7. oder 8. Nebengruppe des Periodensystems der Elemente, ein Element der Lanthanoiden und/oder Actinoiden und ein oder mehrere N-Phenylpyrrolbisphosphane der Formel I 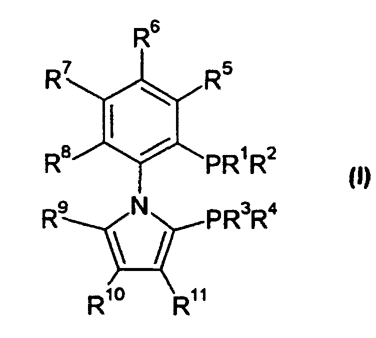 mit
R¹, R², R³, R⁴ = aliphatischer, cycloaliphatischer oder aromatischer Kohlenwasserstoffrest mit 1 bis 25 Kohlenstoffatomen
R⁵, R⁶, R⁷, R⁸, R⁹, R¹⁰, R¹¹ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, wobei R⁵ bis R¹¹ eine gleiche oder unterschiedliche Bedeutung besitzen und kovalent miteinander verknüpft sein können, F, Cl, Br, I, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³, NR¹²R¹³, N⁺R¹²R¹³R¹³, N=CR¹²R¹³, NH₂, wobei R¹² , R¹³ = H, substituierter oder unsubstituierter, aliphatischer oder aromatischer Kohlenwasserstoffrest mit 1 bis 25 Kohlenstoffatomen, mit gleicher oder unterschiedlicher Bedeutung und M = Alkalimetall-, Erdalkalimetall-, Ammonium-, Phosphoniumion.

9. N-Phenylpyrrolbisphosphan-Metallkomplex nach Anspruch 8,
**dadurch gekennzeichnet,**
**dass** R⁵ und R⁶ aus Formel I gemäß Formel II gemeinsam für einen annelierten Aromaten stehen, 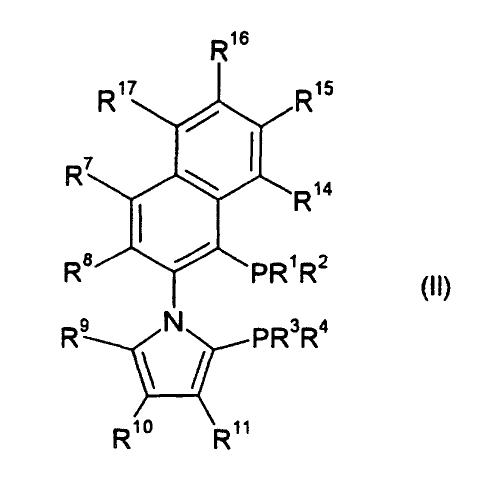 mit
R¹⁴, R¹⁵, R¹⁶, R¹⁷ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, F, Cl, Br, l, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³, NR¹²R¹³, N⁺R¹²R¹³R¹³, N=CR¹²R¹³, NH₂, wobei R¹², R¹³= H, substituierter oder unsubstituierter, aliphatischer oder aromatischer Kohlenwasserstoffrest mit 1 bis 25 Kohlenstoffatomen, mit gleicher oder unterschiedlicher Bedeutung und M = Alkalimetall-, Erdalkalimetall-, Ammonium-, Phosphoniumion und R¹, R², R³, R⁴, R⁷, R⁸, R⁹, R¹⁰ und R¹¹ die in Anspruch 7 genannten Bedeutungen besitzen.

10. N-Phenylpyrrolbisphosphan-Metallkomplex nach Anspruch 8,
**dadurch gekennzeichnet,**
**dass** R⁶ und R⁷ aus Formel I gemäß Formel III gemeinsam für einen annelierten Aromaten stehen, 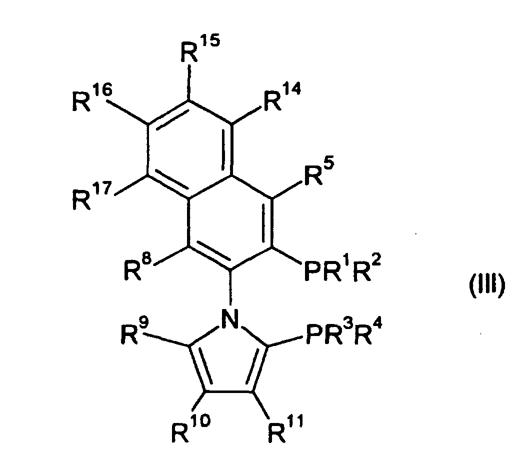 mit
R¹⁴, R¹⁵, R¹⁶, R¹⁷ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, F, Cl, Br, l, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³, NR¹²R¹³, N+R12R13R13, N=CR¹²R¹³, NH₂, wobei R¹², R¹³ = H, substituierter oder unsubstituierter, aliphatischer oder aromatischer Kohlenwasserstoffrest mit 1 bis 25 Kohlenstoffatomen, mit gleicher oder unterschiedlicher Bedeutung und M = Alkalimetall-, Erdalkalimetall-, Ammonium-, Phosphoniumion und R¹, R², R³, R⁴, R⁵, R⁸, R⁹, R¹⁰ und R¹¹ die in Anspruch 7 genannten Bedeutungen besitzen.

11. N-Phenylpyrrolbisphosphan-Metallkomplex nach Anspruch 8,
**dadurch gekennzeichnet,**
**dass** R⁷ und R⁸ aus Formel I gemäß Formel IV gemeinsam für einen annelierten Aromaten stehen, 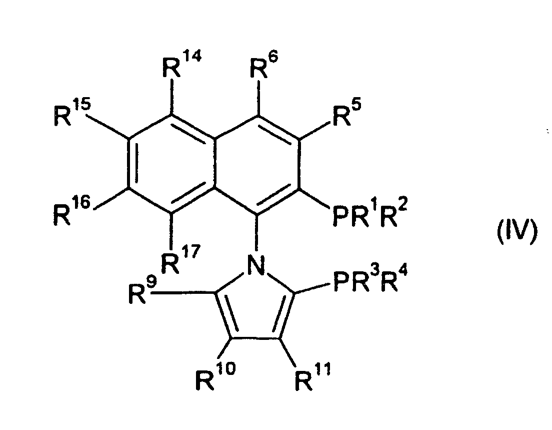 mit
R¹⁴, R¹⁵, R¹⁶, R¹⁷ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, F, Cl, Br, I, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³, NR¹²R¹³, N⁺R¹²R¹³R¹³, N=CR¹²R¹³, NH₂, wobei R¹², R¹³ = H, substituierter oder unsubstituierter, aliphatischer oder aromatischer Kohlenwasserstoffrest mit 1 bis 25 Kohlenstoffatomen, mit gleicher oder unterschiedlicher Bedeutung und M = Alkalimetall-, Erdalkalimetall-, Ammonium-, Phosphoniumion und R¹, R², R³, R⁴, R⁵, R⁶, R⁹, R¹⁰ und R¹¹ die in Anspruch 7 genannten Bedeutungen besitzen.

12. N-Phenylpyrrolbisphosphan-Metallkomplex nach Anspruch 8,
**dadurch gekennzeichnet,**
**dass** R⁹ und R¹⁰ aus Formel I gemäß Formel V gemeinsam für einen annelierten Aromaten stehen, 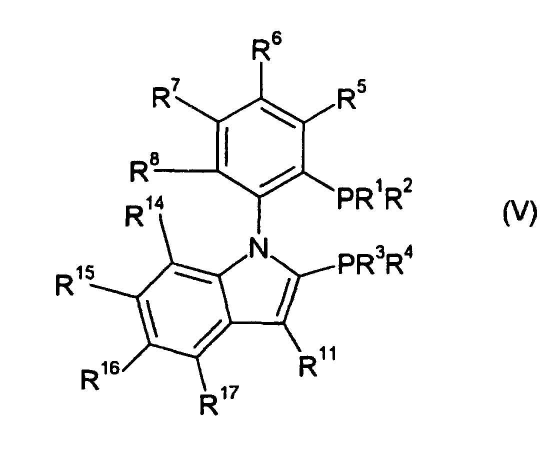 mit
R¹⁴, R¹⁵, R¹⁶, R¹⁷ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, F, Cl, Br, I, -Si(CH₃)₃, -CF₃, -OR¹², -COR¹², -CO₂R¹², -CO₂M, -SR¹² -SO₂R¹², -SOR¹², -SO₃R¹², -SO₃M, -SO₂NR¹²R¹³, NR¹²R¹³, N⁺R¹²R¹³R¹³, N=CR¹²R¹³, NH₂, wobei R¹², R¹³= H, substituierter oder unsubstituierter, aliphatischer oder aromatischer Kohlenwasserstoffrest mit 1 bis 25 Kohlenstoffatomen, mit gleicher oder unterschiedlicher Bedeutung und M = Alkalimetall-, Erdalkalimetall-, Ammonium-, Phosphoniumion und R¹, R², R³, R⁴, R⁵, R⁶, R⁷, R⁸ und R¹¹ die in Anspruch 7 genannten Bedeutungen besitzen.

13. N-Phenylpyrrolbisphosphan-Metallkompex nach einem der Ansprüche 8 bis 12,
**dadurch gekennzeichnet,**
**dass** einer oder mehrere der im Komplex enthaltenen N-Phenylpyrrolbisphosphanliganden chiral ist.

14. N-Phenylpyrrolbisphosphan-Metallkomplex nach einem der Ansprüche 8 bis 13,
**dadurch gekennzeichnet,**
**dass** einer oder mehrere der Reste R¹-R⁴ oder R¹ und R² bzw. R³ und R⁴ von einem oder mehreren im Komplex enthaltenen N-Phenylpyrrolbisphosphanliganden gemeinsam für einen chiralen Rest aus der Gruppe Menthyl, Camphyl, 1,1'-Binaphth-2-yl Hexan-2,5-diyl stehen.

15. N-Phenylpyrrolbisphosphan-Metallkomplex nach einem der Ansprüche 8 bis 14,
**dadurch gekennzeichnet,**
**dass** als Metall ein Metall der 8. Nebengruppe des Periodensystems eingesetzt wird.

16. Verwendung der N-Phenylpyrrolbisphosphane gemäß einem der Ansprüche 1 bis 8 in Hydrierungen, isomerisierungen, Carbonylierungen, Carboxylierungen, Hydroformylierungen, Hydrocyanierungen, Cyclopropanierungen, C-C-Kupplungen, Oligomerisierungen oder Polymerisationen.

17. Verwendung der N-Phenylpyrrolbisphosphan-Metallkomplexe gemäß einem der Ansprüche 8 bis 16 zur Hydrierungen, Isomerisierungen, Carbonylierungen, Carboxylierungen, Hydroformylierungen, Hydrocyanierungen, Cyclopropanierungen, C-C-Kupplungen, Oligomerisierungen oder Polymerisationen.

## Claims

1. N-phenylpyrrolebisphosphane of the formula I 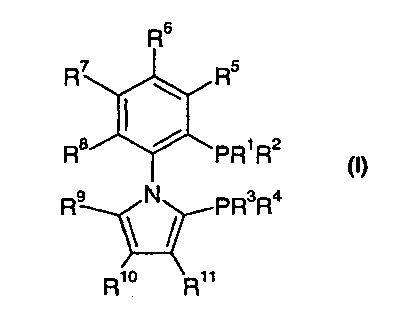 where
R¹, R², R³, R⁴ = aliphatic, cycloaliphatic or aromatic hydrocarbon radicals having from 1 to 25 carbon atoms, and R¹ and R² and/or R³ and R⁴ may be joined by one or more covalent bonds,
R⁵, R⁶, R⁷, R⁸, R⁹, R¹⁰, R¹¹ = H, aliphatic, alicyclic, aliphatic-alicyclic, heterocyclic, aliphatic-heterocyclic, aromatic, aromatic-aromatic, aliphatic-aromatic hydrocarbon radicals having from 1 to 50 carbon atoms, and R⁵ to R¹¹ may each be defined identically or differently and be covalently joined together, and each be F, Cl, Br, I, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³, NR¹²R¹³, N⁺R¹²R¹³R¹³, N=CR¹²R¹³, NH₂, where R¹², R¹³ = H, substituted or unsubstituted, aliphatic or aromatic hydrocarbon radicals having from 1 to 25 carbon atoms, each defined identically or differently, and M = alkali metal, alkaline earth metal, ammonium, phosphonium ion.

2. N-phenylpyrrolebisphosphane according to claim 1,
**characterized in that**
R⁵ and R⁶ from formula I together are a fused aromatic in formula II 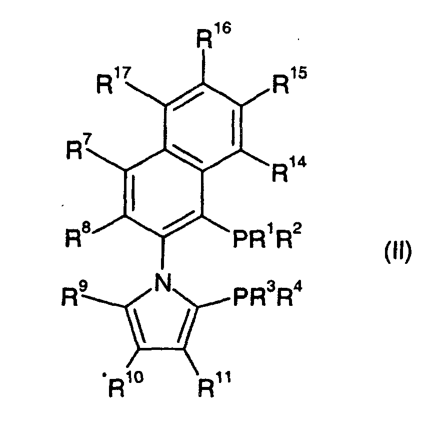 where
R¹⁴, R¹⁵, R¹⁶, R¹⁷ = H, aliphatic, alicyclic, aliphatic-alicyclic, heterocyclic, aliphatic-heterocyclic, aromatic, aromatic-aromatic, aliphatic-aromatic hydrocarbon radicals having from 1 to 50 carbon atoms, and are each F, Cl, Br, I, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³, NR¹²R¹³, N⁺R¹²R¹³R¹³, N=CR¹²R¹³, NH₂, where R¹², R¹³ = H, substituted or unsubstituted, aliphatic or aromatic hydrocarbon radicals having from 1 to 25 carbon atoms, each defined identically or differently, and M = alkali metal, alkaline earth metal, ammonium, phosphonium ion and R¹, R², R³, R⁴, R⁷, R⁸, R⁹, R¹⁰ and R¹¹ are each as defined in claim 1.

3. N-phenylpyrrolebisphosphane according to claim 1,
**characterized in that**
R⁶ and R⁷ from formula I together are a fused aromatic in formula III 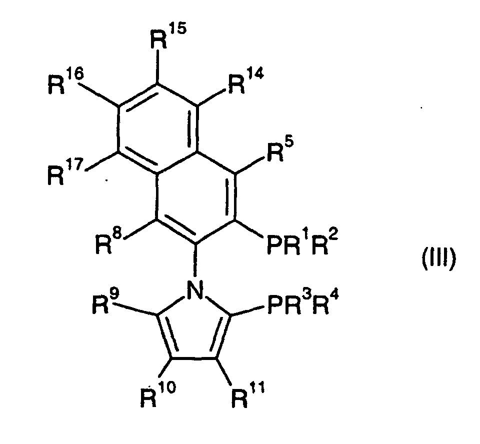 where
R¹⁴, R¹⁵, R¹⁶, R¹⁷*=* H, aliphatic, alicyclic, aliphatic-alicyclic, heterocyclic, aliphatic-heterocyclic, aromatic, aromatic-aromatic, aliphatic-aromatic hydrocarbon radicals having from 1 to 50 carbon atoms, and are each F, Cl, Br, I, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³, NR¹²R¹³, N⁺R¹²R¹³ R¹³, N=CR¹²R¹³, NH₂, where R¹², R¹³ = H, substituted or unsubstituted, aliphatic or aromatic hydrocarbon radicals having from 1 to 25 carbon atoms, each defined identically or differently, and M = alkali metal, alkaline earth metal, ammonium, phosphonium ion and R¹, R², R³, R⁴, R⁵, R⁸, R⁹, R¹⁰ and R¹¹ are each as defined in claim 1.

4. N-phenylpyrrolebisphosphane according to claim 1,
**characterized in that**
R⁷ and R⁸ from formula I together are a fused aromatic in formula IV 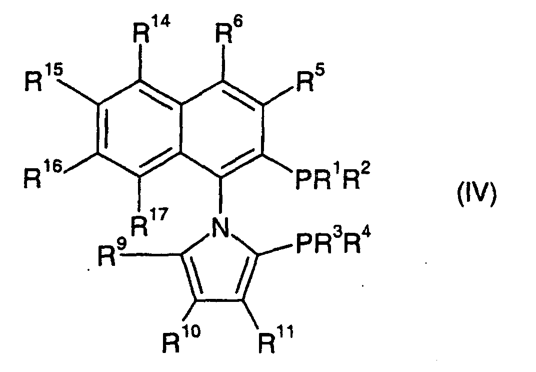 where
R¹⁴, R¹⁵, R¹⁶, R¹⁷ = H, aliphatic, alicyclic, aliphatic-alicyclic, heterocyclic, aliphatic-heterocyclic, aromatic, aromatic-aromatic, aliphatic-aromatic hydrocarbon radicals having from 1 to 50 carbon atoms, and are each F, Cl, Br, I, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³, NR¹²R¹³, N⁺R¹²R¹³R¹³, N=CR¹²R¹³, NH₂, where R¹², R¹³ = H, substituted or unsubstituted, aliphatic or aromatic hydrocarbon radicals having from 1 to 25 carbon atoms, each defined identically or differently, and M = alkali metal, alkaline earth metal, ammonium, phosphonium ion and R¹, R², R³, R⁴, R⁵, R⁶, R⁹, R¹⁰ and R¹¹ are each as defined in claim 1.

5. N-phenylpyrrolebisphosphane according to claim 1,
**characterized in that**
R⁹ and R¹⁰ from formula I together are a fused aromatic in formula V 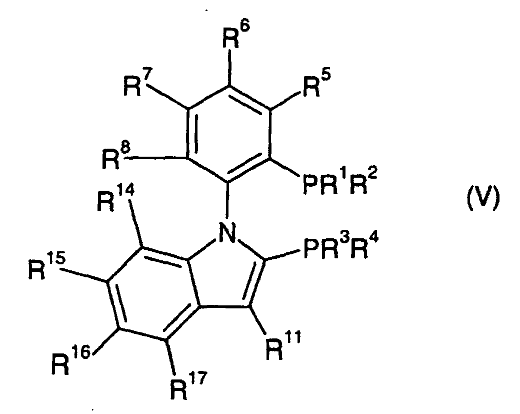 where
R¹⁴, R¹⁵, R¹⁶, R¹⁷ = H, aliphatic, alicyclic, aliphatic-alicyclic, heterocyclic, aliphatic-heterocyclic, aromatic, aromatic-aromatic, aliphatic-aromatic hydrocarbon radicals having from 1 to 50 carbon atoms, and are each F, Cl, Br, I, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³, NR¹²R¹³, N⁺R¹²R¹³R¹³, N=CR¹²R¹³, NH₂, where R¹², R¹³ = H, substituted or unsubstituted, aliphatic or aromatic hydrocarbon radicals having from 1 to 25 carbon atoms, each defined identically or differently, and M = alkali metal, alkaline earth metal, ammonium, phosphonium ion and R¹, R², R³, R⁴, R⁵, R⁶, R⁷, R⁸ and R¹¹ are each as defined in claim 1.

6. N-phenylpyrrolebisphosphane according to any one of claims 1 to 5,
**characterized in that**
the N-phenylpyrrolebisphosphane is chiral.

7. N-phenylpyrrolebisphosphane according to any one of claims 1 to 6,
**characterized in that**
one or more of the radicals R¹-R⁴ or R¹ and R² and/or R³ and R⁴ together are a chiral radical from the group of menthyl, camphyl, 1,1'-binaphth-2-yl, hexane-2,5-diyl.

8. N-phenylpyrrolebisphosphane-metal complex containing a metal of the 1 st, 2nd, 3rd, 4th, 5th, 6th, 7th or 8th transition group of the Periodic Table of the Elements, an element from the lanthanides and/or actinides and one or more N-phenylpyrrolebisphosphanes of the formula I 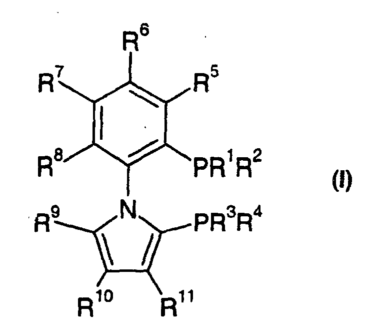 where
R¹, R², R³, R⁴ = aliphatic, cycloaliphatic or aromatic hydrocarbon radicals having 1 to 25 carbon atoms,
R⁵, R⁶, R⁷, R⁸, R⁹, R¹⁰, R¹¹ = H, aliphatic, alicyclic, aliphatic-alicyclic, heterocyclic, aliphatic-heterocyclic, aromatic, aromatic-aromatic, aliphatic-aromatic hydrocarbon radicals having from 1 to 50 carbon atoms, and R⁵ to R¹¹ may each be defined identically or differently and be covalently joined together, and each be F, Cl, Br, I, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³, NR¹²R¹³, N⁺R¹²R¹³R¹³, N=CR¹²R¹³, NH₂, where R¹², R¹³ = H, substituted or unsubstituted, aliphatic or aromatic hydrocarbon radicals having from 1 to 25 carbon atoms, each defined identically or differently, and M = alkali metal, alkaline earth metal, ammonium, phosphonium ion.

9. N-phenylpyrrolebisphosphane-metal complex according to claim 8,
**characterized in that**
R⁵ and R⁶ from formula I together are a fused aromatic in formula II 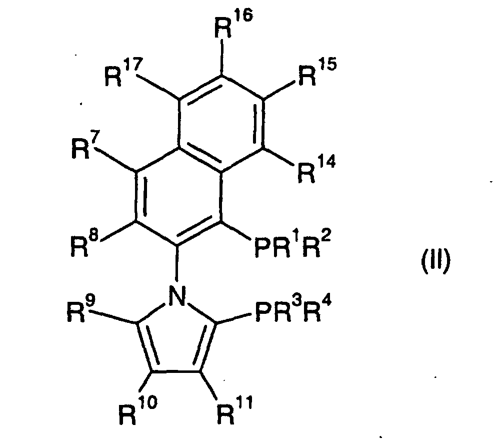 where
R¹⁴, R¹⁵, R¹⁶, R¹⁷ = H, aliphatic, alicyclic, aliphatic-alicyclic, heterocyclic, aliphatic-heterocyclic, aromatic, aromatic-aromatic, aliphatic-aromatic hydrocarbon radicals having from 1 to 50 carbon atoms, and are each F, Cl, Br, I, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³, NR¹²R¹³, N⁺R¹²R¹³R¹³, N=CR¹²R¹³, NH₂, where R¹², R¹³ = H, substituted or unsubstituted, aliphatic or aromatic hydrocarbon radicals having from 1 to 25 carbon atoms, each defined identically or differently, and M = alkali metal, alkaline earth metal, ammonium, phosphonium ion and R¹, R², R³, R⁴, R⁷, R⁸, R⁹, R¹⁰ and R¹¹ are each as defined in claim 7.

10. N-phenylpyrrolebisphosphane-metal complex according to claim 8,
**characterized in that**
R⁶ and R⁷ from formula I together are a fused aromatic in formula III 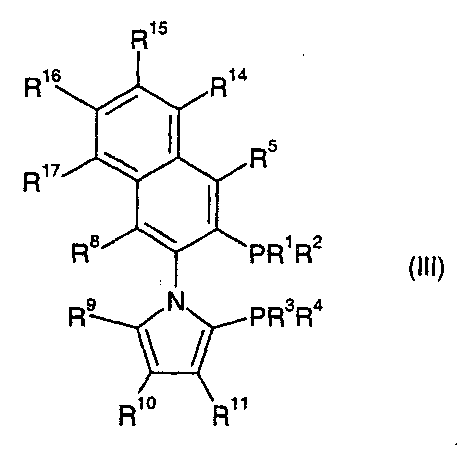 where
R¹⁴, R¹⁵, R¹⁶, R¹⁷ = H, aliphatic, alicyclic, aliphatic-alicyclic, heterocyclic, aliphatic-heterocyclic, aromatic, aromatic-aromatic, aliphatic-aromatic hydrocarbon radicals having from 1 to 50 carbon atoms, and are each F, Cl, Br, I, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³, NR¹²R¹³, N⁺R¹²R¹³R¹³, N=CR¹²R¹³, NH₂, where R¹², R¹³ = H, substituted or unsubstituted, aliphatic or aromatic hydrocarbon radicals having from 1 to 25 carbon atoms, each defined identically or differently, and M = alkali metal, alkaline earth metal, ammonium, phosphonium ion and R¹, R², R³, R⁴, R⁵, R⁸, R⁹, R¹⁰ and R¹¹ are each as defined in claim 7.

11. N-phenylpyrrolebisphosphane-metal complex according to claim 8,
**characterized in that**
R⁷ and R⁸ from formula I together are a fused aromatic in formula IV 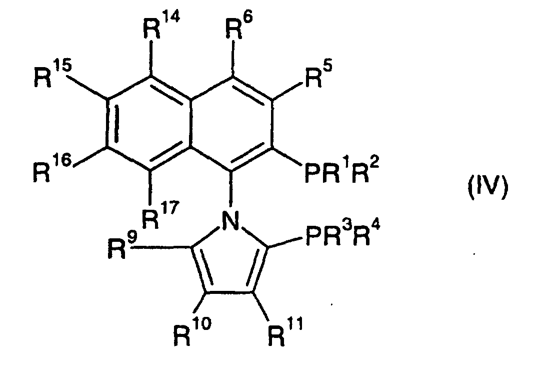 where
R¹⁴, R¹⁵, R¹⁶, R¹⁷= H, aliphatic, alicyclic, aliphatic-alicyclic, heterocyclic, aliphatic-heterocyclic, aromatic, aromatic-aromatic, aliphatic-aromatic hydrocarbon radicals having from 1 to 50 carbon atoms, and are each F, Cl, Br, I, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³, NR¹²R¹³, N⁺R¹²R¹³R¹³, N=CR¹²R1¹³, NH₂, where R¹², R¹³ = H, substituted or unsubstituted, aliphatic or aromatic hydrocarbon radicals having from 1 to 25 carbon atoms, each defined identically or differently, and M = alkali metal, alkaline earth metal, ammonium, phosphonium ion and R¹, R², R³, R⁴, R⁵, R⁶, R⁹, R¹⁰ and R¹¹ are each as defined in claim 7.

12. N-phenylpyrrolebisphosphane-metal complex according to claim 8,
**characterized in that**
R⁹ and R¹⁰ from formula I together are a fused aromatic in formula V 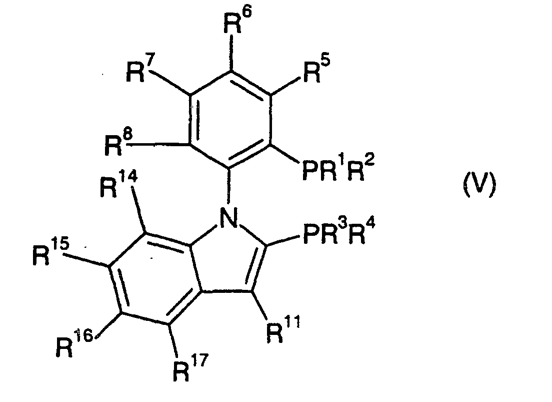 where
R¹⁴, R¹⁵, R¹⁶, R¹⁷ = H, aliphatic, alicyclic, aliphatic-alicyclic, heterocyclic, aliphatic-heterocyclic, aromatic, aromatic-aromatic, aliphatic-aromatic hydrocarbon radicals having from 1 to 50 carbon atoms, and are each F, Cl, Br, I, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³, NR¹²R¹³, N⁺R¹²R¹³R¹³, N=CR¹²R¹³, NH₂, where R¹², R¹³ = H, substituted or unsubstituted, aliphatic or aromatic hydrocarbon radicals having from 1 to 25 carbon atoms, each defined identically or differently, and M = alkali metal, alkaline earth metal, ammonium, phosphonium ion and R¹, R², R³, R⁴, R⁵, R⁶, R⁷, R⁸ and R¹¹ are each as defined in claim 7.

13. N-phenylpyrrolebisphosphane-metal complex according to any one of claims 8 to 12,
**characterized in that**
one or more of the N-phenylpyrrolebisphosphane ligands present in the complex is chiral.

14. N-phenylpyrrolebisphosphane-metal complex according to any one of claims 8 to 13,
**characterized in that**
one or more of the radicals R¹-R⁴ or R¹ and R² and/or R³ and R⁴ of one or more N-phenylpyrrolebisphosphane ligands present in the complex together are a chiral radical from the group of menthyl, camphyl, 1,1'-binaphth-2-yl, hexane-2,5-diyl.

15. N-phenylpyrrolebisphosphane-metal complex according to any one of claims 8 to 14,
**characterized in that**
the metal used is a metal of the 8th transition group of the Periodic Table.

16. Use of an N-phenylpyrrolebisphosphane according to any one of claims 1 to 8 in hydrogenations, isomerizations, carbonylations, carboxylations, hydroformylations, hydrocyanations, cyclopropanations, C-C couplings, oligomerizations or polymerizations.

17. Use of an N-phenylpyrrolebisphosphane-metal complex according to any one of claims 8 to 16 for hydrogenations, isomerizations, carbonylations, carboxylations, hydroformylations, hydrocyanations, cyclopropanations, C-C couplings, oligomerizations or polymerizations.

## Revendications

1. N-phénylpyrrolbisphosphanes repondant à la formule générale 1 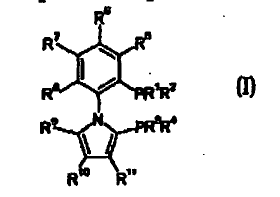 dans laquelle
R¹, R², R³, R⁴ = reste d'hydrocarbure aliphatique, cycloaliphatique ou aromatique comportant de 1 à 25 atomes de carbone. R¹ et R², ou R³ et R⁴ pouvant être reliés par une ou plusieurs liaisons covalentes,
R⁵, R⁶, R⁷, R⁸, R⁹, R¹⁰, R¹¹ = H, reste d'hydrocarbure aliphatique, alicyclique, aliphatique-alicyclique, hétérocyclique, aliphatique-hétérocyclique, aromatique, aromatique-aromatique, aliphatique-aromatique comportant de 1 à 50 atomes de carbone, où R⁵ à R¹¹ possèdent une signification identique ou différente et peuvent être reliés l'un avec l'autre de manière covalente, F, Cl, Br, I, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³, NR¹²R¹³, N⁺R¹²R¹³R¹³, N=CR¹²R¹³, NH₂, où R¹², R¹³ = H, reste d'hydrocarbure aliphatique ou aromatique, substitué ou non substitué, comportant de 1 à 25 atomes de carbone, avec une signification identique ou différente, et M = ion de métal alcalin, de métal alcalinoterreux, d'ammonium, de phosphonium.

2. N-phénylpyrrolbisphosphanes selon la revendication 1,
**caractérisée en ce que**
R⁵ et R⁶ de la formule I représentent, selon la formule II, conjointement un aromate annelé, 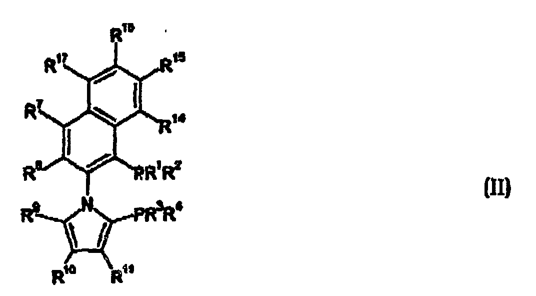 où
R¹⁴, R¹⁵, R¹⁶, R¹⁷ = H, reste d'hydrocarbure aliphatique, alicyclique, aliphatique-alicyclique, hëtérocyclique, aliphatique-hétérocyclique, aromatique, aromatique-aromatique, aliphatique-aromatique comportant de 1 à 50 atomes de carbone, F, Cl, Br, I, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³, NR¹²R¹³, N⁺R¹²R¹³R¹³, N=CR¹²R¹³, NH₂, où R¹², R¹³ = H, reste d'hydrocarbure aliphatique ou aromatique, substitué ou non substitué, comportant de 1 à 25 atomes de carbone, avec une signification identique ou différente, et M = ion de métal alcalin, de métal alcalinoterreux, d'ammonium, de phosphonium, et R¹, R², R³, R⁴, R⁷, R⁸, R⁹, R¹⁰ et R¹¹ possèdent les significations indiquées dans la revendication 1.

3. N-phénylpvyrrolbisphosphanes selon la revendication 1,
**caractérisés en ce que**
R⁶ et R⁷ de la formule I représentent, selon la formule III, conjointement un aromate annelé. 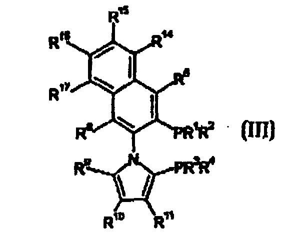 où
R¹⁴, R¹⁵, R¹⁶, R¹⁷ = H, reste d'hydrocarbure aliphatique, alicyclique, aliphatique-alicyclique, hétérocyclique, aliphatique-hétérocyclique, aromatique, aromatique-aromatique, aliphatique-aromatique comportant de 1 à 50 atomes de carbone, F, Cl, Br, I, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³, NR¹²R¹³, N+R¹²R¹³R¹³, N=CR¹²R¹³, NH₂, où R¹², R¹³ = H, reste d'hydrocarbure aliphatique ou aromatique, substitué ou non substitué, comportant de 1 à 25 atomes de carbone, avec une signification identique ou différente, et M = ion de métal alcalin, de métal alcalinoterreux, d'ammonium. de phosphonium, et R¹, R², R³, R⁴, R⁵, R⁸, R⁹, R¹⁰ et R¹¹ possèdent les significations indiquées dans la revendication 1.

4. N-phénylpyrrolbisphosphanes selon la revendication 1.
**caractérisés en ce que**
R⁷ et R⁸ de la formule 1 représentent, selon la formule IV, conjointement un aromate annelé. 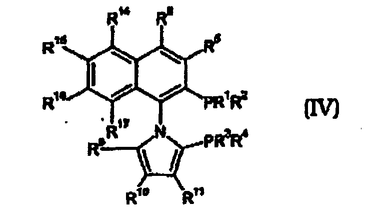 où
R¹⁴, R¹⁵, R¹⁶, R¹⁷ = H, reste d'hydrocarbure aliphatique, alicyclique, aliphatique-alicyclique, hétérocyclique, aliphatique-hétérocyclique, aromatique, aromatlque-aromatique, aliphatique-aromatique comportant de 1 à 50 atomes de carbone, F, Cl, Br, I, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³, NR¹²R¹³, N⁺R¹²R¹³R¹³, N=CR¹²R¹³, NH₂, où R¹², R¹³ = H, reste d'hydrocarbure aliphatique ou aromatique, substitué ou non substitué, comportant de 1 à 25 atomes de carbone, avec une signification identique ou différente, et M = ion de métal alcalin, de métal alcalinoterreux. d'ammonium, de phosphonium, et R¹, R², R³, R⁴, R⁵, R⁶, R⁹, R¹⁰ et R¹¹ possèdent les significations indiquées dans la revendication 1.

5. N-phénylpyrrolbisphosphanes selon la revendication 1,
**caractérisés en ce que**
R⁹ et R¹⁰ de la formule I représentent, selon la formule V, conjointement un aromate annelé, 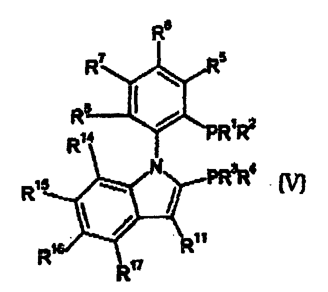 où
R¹⁴, R¹⁵, R¹⁶, R¹⁷ = H, reste d'hydrocarbure aliphatique, alicyclique, aliphatique-alicyclique, hétérocyclique, aliphatique-hétérocyclique, aromatique, aromatique-aromatique, aliphatique-aromatique comportant de 1 à 50 atomes de carbone, F, Cl, Br, I, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³, NR¹²R¹³, N⁺R¹²R¹³R¹³, N=CR¹²R¹³, NH₂, où R¹², R¹³ = H, reste d'hydrocarbure aliphatique ou aromatique, substitué ou non substitué, comportant de 1 à 25 atomes de carbone, avec une signification identique ou différente, et M = ion de métal alcalin, de métal alcalinoterreux, d'ammonium, de phosphonium, et R¹, R², R³, R⁴, R⁵, R⁶, R⁷, R⁸ et R¹¹ possèdent les significations indiquées dans la revendication 1.

6. N-phénylpyrrolbisphosphanes selon l'une des revendications 1 à 5,
**caractérisés en ce que**
les N-phénylpyrrolbisplaosphanes sont chiraux.

7. N-phénylpyrrolbisphosphanes selon l'une des revendications 1 à 6,
**caractérisés en ce que**
un ou plusieurs des restes R¹- R⁴ ou R¹ et R² ou R³ et R⁴ représentent conjointement un reste chiral du groupe menthyle, camphyle, 1, 1'-binapht-2-yle, hexan-2,5-diyle.

8. Complexe métallique de N-phénylpyrrolbisphosphane contenant un métal du 1^{er}, 2^{ème}, 3^{ème}, 4^{ème}, 5^{ème}, 6^{ème}, 7^{ème} ou 8^{ème} sous-groupe du tableau périodique des éléments, un élément des lanthanoïdes et/ou actinoides et un ou plusieurs N-phénylpyrrolbisphosphanes répondant à la formule I 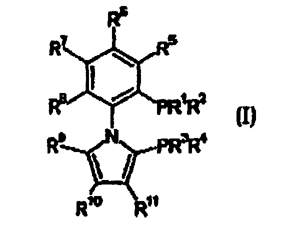 ou
R¹, R², R³, R⁴ = reste d'hydrocarbure aliphatique, cycloaliphatique ou aromatique comportant de 1 à 25 atomes de carbone,
R⁵, R⁶, R⁷, R⁸, R⁹, R¹⁰, R¹¹ = H, reste d'hydrocarbure aliphatique, alicyclique, aliphatique-alicyclique, héterocyclique, aliphatique-hétérocyclique, aromatique, aromatique-aromatique, aliphatique-aromatique comportant de 1 à 50 atomes de carbone, où R⁵ à R¹¹ possèdent une signification identique ou différente et peuvent être reliés l'un avec l'autre de manière covalente, F, Cl, Br, I, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³, NR¹²R¹³, N⁺R¹²R¹³R¹³, N=CR¹²R¹³, NH₂, où R¹², R¹³ = H, reste d'hydrocarbure aliphatique ou aromatique, substitué ou non substitué, comportant de 1 à 25 atomes de carbone, avec une signification identique ou différente, et M = ion de métal alcalin, de métal alcalinoterreux, d'ammonium, de phosphonium.

9. Complexe métallique de N-phénylpyrrolbisphosphane selon la revendication 8,
**caractérisé en ce que**
R⁵ et R⁶ de la formule I représentent, selon la formule II, conjointement un aromate annelé, 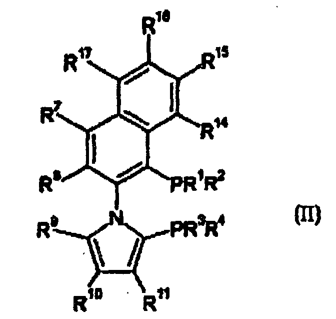 où
R¹⁴, R¹⁵, R¹⁶, R¹⁷ = H, reste d'hydrocarbure aliphatique, alicyeltque, aliphatique-alicyclique, hétérocyclique, aliphatique-hétérocyclique, aromatique, aromatique-aromatique, aliphatique-aromatique comportant de 1 à 50 atomes de carbone, F, CI. Br, I, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³, NR¹²R¹³, N⁺R¹²R¹³R¹³, N=CR¹²R¹³, NH₂, où R¹², R¹³ = H. reste d'hydrocarbure aliphatique ou aromatique, substitué ou non substitué, comportant de 1 à 25 atomes de carbone, avec une signification identique ou différente, et M = ion de métal alcalin, de métal alcalinoterreux, d'ammonium, de phosphonium, et R¹, R², R³, R⁴, R⁷, R⁸, R⁹, R¹⁰ et R¹¹ possèdent les significations indiquées dans la revendication 7.

10. Complexe métallique de N-phénylpyrrolbisphosphane selon la revendication 8,
**caractérisé en ce que**
R⁶ et R⁷ de la formule I représentent, selon la formule III, conjointement un aromate annelé, 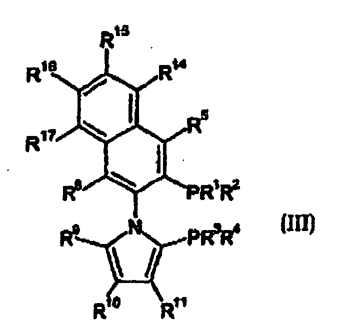 où
R¹⁴, R¹⁵, R¹⁶, R¹⁷ = H, reste d'hydrocarbure aliphatique, alicyclique, aliphatique-alicyclique, hétérocyclique, aliphatique-hétérocyclique, aromatique, aromatique-aromatique, aliphatique-aromatique comportant de 1 à 50 atomes de carbone, F, Cl, Br, I, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³, NR¹²R¹³, N⁺R¹²R¹³R¹³, N=CR¹²R¹³, NH₂, où R¹², R¹³ = H, reste d'hydrocarbure aliphatique ou aromatique, substitué ou non substitué, comportant de 1 à 25 atomes de carbone, avec une signification identique ou différente, et M = ion de métal alcalin, de métal alcalinoterreux. d'ammonium, de phosphonium, et R¹, R², R³, R⁴, R⁵, R⁸, R⁹, R¹⁰ et R¹¹ possèdent les significations indiquées dans la revendication 7.

11. Complexe métallique de N-phénylpyrrolbisphosphane selon la revendication 8.
**caractérisé en ce que**
R⁷ et R⁸ de la formule I représentent, selon la formule IV, conjointement un aromate annelé. 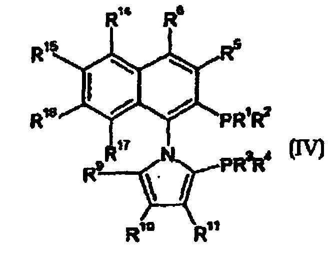 R¹⁴, R¹⁵, R¹⁶, R¹⁷ = H, reste d'hydrocarbure aliphatique, alicyclique, aliphatique-alicyclique, hétérocyclique. aliphatique-hétérocyclique, aromatique, aromatique-aromatique, aliphatique-aromatique comportant de 1 à 50 atomes de carbone, F. Cl, Br, I, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³, NR¹²R¹³, N⁺R¹²R¹³R¹³, N=CR¹²R¹³, NH₂, où R¹², R¹³ = H, reste d'hydrocarbure aliphatique ou aromatique, substitué ou non substitué, comportant de 1 à 25 atomes de carbone, avec une signification identique ou différente, et M = ion de métal alcalin, de métal alcalinoterreux, d'ammonium, de phosphonium, et R¹, R², R³, R⁴, R⁵, R⁶, R⁹, R¹⁰ et R¹¹ possèdent les significations indiquées dans la revendication 7.

12. Complexe métallique de N-phénylpyrrolbisphosphane selon la revendication 8,
**caractérisé en ce que**
R9 et R¹⁰ de la formule I représentent, selon la formule V, conjointement un aromate annelé, 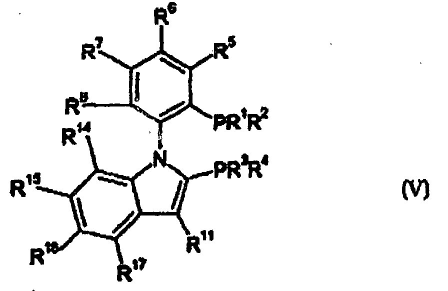 où
R¹⁴, R¹⁵, R¹⁶, R¹⁷ = H, reste d'hydrocarbure aliphatique, alicyclique, aliphatique-alicyclique, hétérocyclique, aliphatique-hétérocyclique, aromatique, aromatique-aromatique. aliphatique-aromatique comportant de 1 à 50 atomes de carbone, F, Cl, Br, I, -Si(CH₃)₃, -CF₃, -OR¹², -COR¹², -CO₂R¹², -CO₂M, -SR¹², -SO₂R¹², -SOR¹², -SO₃R¹², -SO₃M, -SO₂NR¹²R¹³, NR¹²R¹³, N+R¹²R¹³R¹³, N=CR¹²R¹³, NH₂, où R¹², R¹³ = H, reste d'hydrocarbure aliphatique ou aromatique, substitué ou non substitué, comportant de 1 à 25 atomes de carbone, avec une signification identique ou différente, et M = ion de métal alcalin, de métal alcalinoterreux, d'ammonium, de phosphonium, et R¹, R², R³, R⁴, R⁵, R⁶, R⁷, R⁸ et R¹¹ possèdent les significations indiquées dans la revendication 7.

13. Complexe métallique de N-phénylpyrrolbisphosphane selon l'une des revendications 8 à 12,
**caractérisé en ce qu'**
un ou plusieurs des ligands N-phénylpyrrolbisphosphanes contenus dans le complexe est chiral.

14. Complexe métallique de N-phénylpyrrolbisphosphane selon l'une des revendications 8 à 13,
**caractérisé en ce qu'**
un ou plusieurs des restes R¹- R⁴, ou R¹ et R², ou R³ et R⁴ d'un ou de plusieurs ligands N-phénylpyrrolbisphosphanes contenus dans le complexe représentent conjointement un reste chiral du groupe menthyle, camphyle, 1,1'-binapht-2-yle, hexan-2,5-diyle.

15. Complexe métallique de N-phénylpyrrolbisphosphane selon l'une des revendications 8 à 14,
**caractérisé en ce que**,
en tant que métal, un métal du 8^{ème} sous-groupe du tableau périodique est utilisé.

16. Utilisation des N-phénylpyrrolbisphosphanes selon l'une des revendications 1 à 7 dans les hydrogénations, isomérisations, carbonylations, carboxylations, hydroformylations, hydrocyanations, cyclopropanations. couplages C-C, oligomérisations, ou polymérisations.

17. Utilisation des complexes métalliques de N-phénylpyrrolbisphosphanes selon l'une des revendications 8 à 16 pour les hydrogénations, isomérisations, carbonylations, carboxylations, hydroformylations, hydrocyanations, cyclopropanations, couplages C-C, oligomérisations, ou polymérisations.
